# EUROPEAN PATENT APPLICATION

(11) **EP 4 581 930 A1**
(43) Date of publication of application: **09.07.2025**
(21) Application number: 23860343.5
(22) Date of filing: 29.08.2023
(51) Int. Cl.: A01N 43/42, A01N 43/36, A01N 43/40, A01N 43/84, A01N 43/90, A01P 1/00, A01P 3/00, A61L 9/00, A61L 9/01, D06M 13/388

(54) **INACTIVATION METHOD AND INACTIVATOR FOR BACTERIA OR VIRUSES, AND ANTIVIRAL BASE MATERIAL USING SAME**

(30) Priority: 29.08.2022 JP 2022136098
(71) Applicant: NISSAN MOTOR CO., LTD., Kanagawa 221-0023 (JP); Tohoku University, Sendai-shi, Miyagi 980-8577 (JP)
(72) Inventor: ITO, Masashi, Atsugi-shi, Kanagawa 243-0123 (JP); OUME, Ami, Atsugi-shi, Kanagawa 243-0123 (JP); UCHIMURA, Masanobu, Atsugi-shi, Kanagawa 243-0123 (JP); MATSUMOTO, Kota, Atsugi-shi, Kanagawa 243-0123 (JP); SONE, Kazuki, Atsugi-shi, Kanagawa 243-0123 (JP); HIRASAWA, Noriyasu, Sendai-shi, Miyagi 980-8577 (JP); IWABUCHI, Yoshiharu, Sendai-shi, Miyagi 980-8577 (JP); KONNO, Tomohiro, Sendai-shi, Miyagi 980-8577 (JP); SASANO, Yusuke, Sendai-shi, Miyagi 980-8577 (JP); SEGAWA, Ryosuke, Sendai-shi, Miyagi 980-8577 (JP)
(74) Representative: Grünecker Patent- und Rechtsanwälte PartG mbB
(86) International application number: PCT/JP2023/031213
(87) International publication number: WO 2024/048581

(57) **Abstract**

There is provided inexpensive means capable of exhibiting an inactivation effect on microorganisms without assuming the presence of light while minimizing the burden on the environment.

Bacteria or viruses are inactivated by bringing a compound having a redox mechanism below into contact with a target suspected to be contaminated by bacteria or viruses: 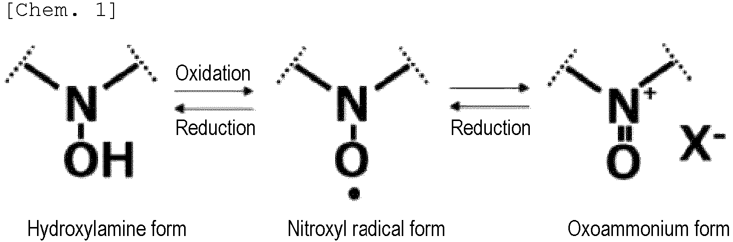 in the formula, X⁻ represents a counteranion, and a broken line represents a bonding position with another atom.

## Description

### TECHNICAL FIELD

The present invention relates to a method and an agent for inactivating bacteria or viruses, and an antiviral base material using the same.

### BACKGROUND ART

Recent globalization has increased the risk of viral outbreaks, and since 1990 infectious diseases such as the avian influenza (A/H5N1) in 1997, the SARS (SARS-Cov) in 2002, the novel influenza (A/4H1N1) in 2009, and the MERS (MERS-Cov) in 2015 have spread worldwide. This is much higher than the frequency of pandemic before 1990. The emergence of the novel coronavirus that caused the pandemic in 2019, and COVID-19 (SARS-Cov-2), which is an acute respiratory infectious disease, not only affect human life but also have a significant impact on the economy, and are a serious threat in terms of protecting the safe and secure living environment of humanity.

Ventilation and introduction of outside air are recommended to remove viruses. However, ventilation and introduction of outside air disturb the temperature and humidity environment adjusted by air conditioning, and thus, from an energy-saving perspective, it is desirable to quickly inactivate viruses by internal air circulation. By using a filter or an interior material having antimicrobial and antiviral properties, inactivation of bacteria and viruses in the internal air circulation can be expected. Here, as a technique for inducing conventional antimicrobial and antiviral properties, a technique for exhibiting antimicrobial and antiviral effects using metal ions has been proposed in Japanese Patent No. 4584339 and Japanese Patent Application Laid-Open No. 2017-133137. Furthermore, as an inorganic oxide system, a system that functions as a photocatalyst has also been proposed.

### SUMMARY OF INVENTION

### Technical Problem

The inorganic metal systems disclosed in Japanese Patent No. 4584339 and Japanese Patent Application Laid-Open No. 2017-133137 exhibit an action by protein denaturation on the premise of sustained release of metal ions, but there is a problem that there is a concern about an adverse effect on the environment. In addition, silver and copper, which are considered to be particularly highly effective, are not only useful as noble metals but also expensive. Further, it is necessary to dissolve and diffuse the metal ions for sustained release of the metal ions, and there is also a problem that it takes time until an antimicrobial and antiviral effect is obtained. In addition, since an inorganic oxide-based (photocatalyst) does not function unless light exists, there is a problem that a light irradiation device is separately required when the inorganic oxide-based (photocatalyst) is used in a situation where light such as sunlight is applied or when the inorganic oxide-based (photocatalyst) is used in a dark place.

Therefore, an object of the present invention is to provide inexpensive means capable of exhibiting an inactivation effect on microorganisms without assuming the presence of light while minimizing the burden on the environment.

### Solution to Problem

The present inventors have conducted intensive studies to solve the above problems. As a result, the present inventors have found that microorganisms can be inactivated by bringing a compound having a predetermined redox mechanism into contact with a target suspected to be contaminated by microorganisms, and have completed the present invention.

That is, one aspect of the present invention is a method for inactivating bacteria or viruses, including bringing a compound having the following redox mechanism into contact with a target suspected to be contaminated by bacteria or viruses.

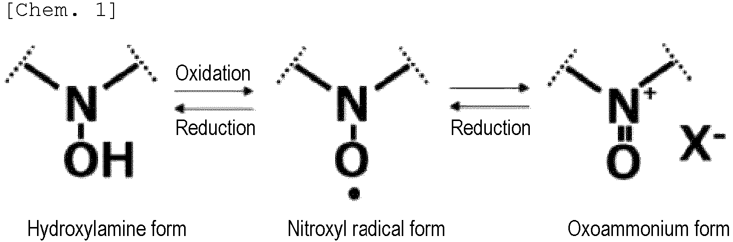

In the formula, X⁻ represents a counteranion, and a broken line represents a bonding position with another atom.

### BRIEF DESCRIPTION OF DRAWINGS

Fig. 1 is a cyclic voltammogram obtained by measuring a standard redox potential (E⁰@25°C) between a nitroxyl radical form and an oxoammonium form by a method using cyclic voltammetry for four compounds of (a) TEMPO, (b) AZADO, (c) 1-methyl AZADO, and (d) 1,3-dimethyl AZADO.
Fig. 2 is a graph showing the results (cell viability) of evaluating the antiviral effect by the WST-8 method after treating a dilution series of culture supernatants of CRFK cells infected with CoV using AZADO-Oxo or silver nitrate for the purpose of confirming the antiviral effect of AZADO.
Fig. 3 is a view showing photographs of wells observed with a microscope for samples obtained by treating culture supernatants of CRFK cells infected with CoV with AZADO-Oxo for the purpose of confirming the antiviral effect of AZADO, together with photographs of cells to which no virus was added (no virus) and cells to which virus was added but AZADO-Oxo was not added (with virus).
Fig. 4 is a graph showing the results of evaluating the antiviral effect (cell viability) by the WST-8 method for samples obtained by treating culture supernatants of CRFK cells infected with CoV using AZADO-Oxo for the purpose of confirming the antiviral effect of AZADO.
Fig. 5 is a graph showing the results of flow cytometry evaluation of S-RBD-His (wild type) or S-RBD-His (Omicron strain) mixed with AZADO-Oxo having different final concentrations in order to confirm whether AZADO-Oxo exhibits antiviral activity by denaturing a spike protein by oxidation to inhibit binding between the spike protein and ACE2. The results for the wild type strain (S-RBD-His(WT)) are shown in (a) of Fig. 5, and the results for the Omicron strain (S-RBD-His(Omicron)) are shown in (b) of Fig. 5.
Fig. 6 is a graph showing the results of mixing S-RBD-His (wild type) with compounds having different final concentrations and evaluating by flow cytometry using AZADO⁺BF₄⁻, TEMPO⁺BF₄⁻, and 4-AcNH-TEMPO⁺BF₄⁻ for the purpose of comparing antiviral effects among compounds. The results for AZADO⁺BF₄⁻ are shown in (a) of Fig. 6, the results for TEMPO⁺BF₄⁻ are shown in (b) of Fig. 6, and the results for 4-AcNH-TEMPO⁺BF₄⁻ are shown in (c) of Fig. 6. In the present specification, "AcNH", "acetylamino", "acetamide", and "NHAc" represent the same functional group.
Fig. 7 is a graph showing the results of graphing a mean value of fluorescence intensity (mean fluorescence intensity) at each peak shown in Fig. 6.
Fig. 8 is a graph showing MFI obtained by performing the same operation as in the experiments shown in Figs. 6 and 7 for AZADO+BF₄⁻ and 4-AcNH-TEMPO⁺BF₄⁻, which showed high antiviral effects, except that the treatment time was extended from 30 minutes to 6 hours when added to S-RBD-His (wild type).
Fig. 9 is an electrophoretic photograph showing the results of CBB staining of a supernatant for samples treated with ovalbumin (OVA) using AZADO/DB, AZADO-Cu(bpy)/DB, Cu(bpy)/DB, and Denka Black (DB) prepared in Examples.
Fig. 10 is an electrophoretic photograph showing the results of CBB staining of a supernatant and a residue for samples treated with ovalbumin (OVA) using AZADO/DB, AZADO-Cu(bpy)/DB, Cu(bpy)/DB, and Denka Black (DB) prepared in Examples.
Fig. 11 is an electrophoretic photograph showing the results of CBB staining of a supernatant for samples treated with ovalbumin (OVA) using unsupported AZADO. Fig. 11 shows the results using AZADO-Oxo.
Fig. 12A is a graph showing the results of calculating the viable cell ratio after plate culture of a bacterial suspension which is in contact with the compounds according to the present invention (TEMPO⁺NO₃⁻, AZADO⁺NO₃⁻, and 4-AcNH-TEMPO⁺NO₃⁻) for the purpose of evaluating the inactivation activity against Escherichia coli.
Fig. 12B is a graph showing the results of calculating the viable cell ratio after plate culture of a bacterial suspension which is in contact with the compounds according to the present invention (TEMPO⁺NO₃⁻, AZADO⁺NO₃⁻, and 4-AcNH-TEMPO⁺NO₃⁻) for the purpose of evaluating the inactivation activity against Staphylococcus aureus.
Fig. 12C is a graph showing the results of calculating the viable cell ratio after plate culture of a bacterial suspension which is in contact with the compounds according to the present invention (TEMPO⁺NO₃⁻, AZADO⁺NO₃⁻, and 4-AcNH-TEMPO⁺NO₃⁻) for the purpose of evaluating the inactivation activity against Penicillium citrinum.
Fig. 13 is a photograph of an agar medium after culturing a sample with a final concentration of AZADO⁺NO₃⁻ was 1 mM when the minimum inhibitory concentration (MIC) against Escherichia coli was measured for the compound (AZADO⁺NO₃⁻) according to the present invention.
Fig. 14 is an electrophoretic photograph showing the results of evaluating the amount of S-RBD after reaction by an SDS-PAGE method and a CBB staining method after reacting the antiviral base material (AZADO-Cu(bpy)/DB) according to the present invention with S-RBD in a liquid phase prior to evaluating the inactivation effect on proteins in a mist state.
Fig. 15 is an electrophoretic photograph showing the results of evaluating the amount of S-RBD remaining on the filter after spraying an aqueous solution containing S-RBD to the filter containing the antiviral base material (AZADO-Cu(bpy)/DB) according to the present invention and incubating, by an SDS-PAGE method and a silver staining method for the purpose of evaluating the inactivation effect on proteins in a mist state.

### DESCRIPTION OF EMBODIMENTS

Hereinafter, embodiments for carrying out the present invention will be described.

### <<Method for Inactivating Bacteria or Viruses>>

That is, one aspect of the present invention is a method for inactivating bacteria or viruses, including bringing a compound (hereinafter also referred to as "compound of the present aspect") having the above redox mechanism into contact with a target suspected to be contaminated by bacteria or viruses. Therefore, according to the present invention, there is provided inexpensive means capable of exhibiting an inactivation effect on microorganisms without assuming the presence of light while minimizing the burden on the environment.

### <Compound of Present Aspect >

The compound of the present aspect is conventionally known as "organic nitroxyl radical". The nitroxyl radical is a stable organic free radical molecule, and is widely used as an oxidation catalyst in the field of organic synthesis. Among them, the organic nitroxyl radical exhibits bidirectional reactivity of oxidation and reduction, and as shown in the above reaction formula, a reaction occurs in which a one-electron reduction generates hydroxylamine, and a reaction occurs in which a one-electron oxidation generates an oxoammonium ion. Oxoammonium ions generated by oxidation of nitroxyl radicals have a strong oxidizing power, and give hydroxylamine after a two-electron oxidation of a substrate. The compound of the present aspect acts as an oxidizing agent on proteins of bacteria and viruses, oxidizes the proteins, and reduces or eliminates the functions thereof. Accordingly, an action of inactivating bacteria and viruses is exhibited. Here, the mass transfer involved in the redox mechanism is only H·, proton (H+), and electron (e⁻), and a reaction faster than the redox of metal ions such as Ag and Cu proposed in the related art occurs, and thus more rapid inactivation of bacteria and viruses can be achieved. In addition, the method for inactivating microorganisms according to the present aspect is based on an oxidation reaction, and since bacteria and viruses are composed of proteins, there is also an advantage that an inactivation action can be exhibited regardless of bacterial species and fungal species, and regardless of the presence or absence of an envelope of virus species, or the like. In the oxidation reaction in which the oxoammonium ion acts as an oxidation active species, a reactant that promotes reoxidation of the hydroxylamine is added to regenerate the oxoammonium ion, and accordingly, the cycle can be completed to function as a catalyst.

The compound of the present aspect may have the above-described redox mechanism, and the specific structure thereof is not limited at all. In addition, the compound of the present aspect may be a hydroxyamine form, a nitroxyl radical form, or an oxoammonium form in the redox mechanism described above. Among them, a hydroxyamine form or a nitroxyl radical form is preferable, and a nitroxyl radical form is particularly preferable.

In addition, from the viewpoint of exhibiting excellent inactivation activity against microorganisms, the standard redox potential (25°C) between the nitroxyl radical form and the oxoammonium form for the compound of the present aspect is preferably +100 mV to +1000 mV [Ag/Ag⁺], and more preferably +130 mV to +600 mV [Ag/Ag⁺]. The standard redox potential is measured by cyclic voltammetry (CV) under a temperature condition of 25°C. At this time, the measurement is performed by a three-electrode method using a glassy carbon electrode (inner diameter: 3 mm) as a working electrode, a reference electrode (Ag/Ag⁺), and a counter electrode (platinum wire), and the sweep rate of the potential is 50 mV/s. It is noted that the hydroxyamine forms of the compounds of the present invention are generally unstable and rapidly oxidized into nitroxyl radical forms. Thus, the value of the standard redox potential measured by the above method is the value between the nitroxyl radical form and the oxoammonium form.

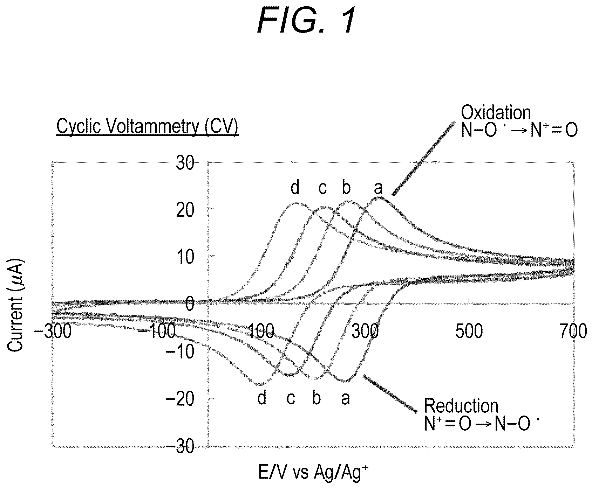

Here, Fig. 1 shows a cyclic voltammogram obtained by measuring the standard redox potential (E⁰@25°C) between the nitroxyl radical form and the oxoammonium form by the method using cyclic voltammetry described above for four compounds of (a) TEMPO, (b) AZADO, (c) 1-methyl AZADO, and (d) 1,3-dimethyl AZADO. As shown in Fig. 1, the E⁰ values for these compounds were (d) 136 mV < (c) 186 mV < (b) 236 mV < (a) 294 mV, respectively. In the cyclic voltammogram shown in Fig. 1, the cyclic voltammogram was stably maintained even after repeating 100 or more cycles of measurement, and thus it is observed that the compound of the present aspect has very high durability as an oxidizing agent.

From the viewpoint of inactivation activity against microorganisms, the nitroxyl radical form of the compound of the present aspect is preferably represented by the following Chemical Formula 1.

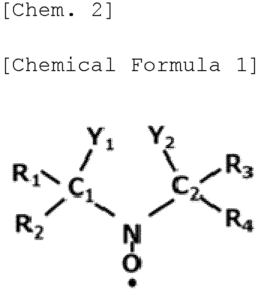

In Chemical Formula 1, R₁ to R₄, Y₁, and Y₂ are each independently a hydrogen atom or an optionally substituted monovalent organic group. Here, the monovalent organic group is not particularly limited, and examples thereof include a substituted or unsubstituted alkyl group, a substituted or unsubstituted alkenyl group, a substituted or unsubstituted alkynyl group, a substituted or unsubstituted aryl group, a substituted or unsubstituted heteroaryl group, and the like. R₁ to R₄, Y₁, and Y₂ may be the same or different.

Each of the alkyl group, the alkenyl group, and the alkynyl group as the monovalent organic group may be linear, branched, or cyclic, but is preferably linear. The number of carbon atoms in the alkyl group is not particularly limited, but is preferably 1 or more and 20 or less, more preferably 1 or more and 10 or less, and still more preferably 1 or more and 4 or less. The number of carbon atoms in each of the alkenyl group and the alkynyl group is not particularly limited, but is preferably 2 or more and 20 or less, more preferably 2 or more and 10 or less, and still more preferably 2 or more and 4 or less.

The aryl group as the monovalent organic group is a group derived from a hydrocarbon ring having aromaticity as a part or the whole. When the aryl group includes two or more hydrocarbon rings having aromaticity as a part or a whole, these rings may be bonded or fused to each other by a single bond. When the aryl group contains two or more hydrocarbon rings having aromaticity as a part or a whole, one atom may also serve as a ring-forming atom of any two of these rings to form a spiro ring. The number of carbon atoms in the aryl group is not particularly limited, but is preferably 6 or more and 30 or less. The number of carbon atoms in the aryl group is more preferably 6 or more and 12 or less, and still more preferably 6. In particular, the aryl group is preferably a monovalent group derived from an aromatic hydrocarbon ring having 6 or more ring-forming atoms.

The heteroaryl group as a monovalent organic group is a group derived from a heterocyclic ring having aromaticity as a part or as a whole. When the heteroaryl group includes two or more heterocyclic rings having aromaticity as a part or a whole, some or all of these rings may be bonded to each other by a single bond. When the heteroaryl group includes two or more heterocyclic rings having aromaticity as a part or a whole, or other rings, these rings may be fused to each other. When the heteroaryl group includes two or more heterocyclic rings having aromaticity as a part or the whole, or other rings, one atom may also serve as a ring-forming atom of these rings. The heteroatom included in the heteroaryl group is not particularly limited, and examples thereof include one or more heteroatoms (for example, a nitrogen atom (N), an oxygen atom (O), a phosphorus atom (P), a sulfur atom (S), a silicon atom (Si), a selenium atom (Se), and a germanium atom (Ge)) and the like as a ring-forming atom. The number of carbon atoms in the heteroaryl group is preferably 3 or more and 30 or less. In addition, the number of ring-forming atoms of the heteroaryl group is not particularly limited, but is preferably 5 or more and 30 or less. Furthermore, the number of ring-forming atoms of the heteroaryl group is more preferably 5 or more and 14 or less, and still more preferably 5 or more and 13 or less. The number of heteroatoms in the heteroaryl group is not particularly limited, but is preferably 1 or more and 3 or less. In addition, the number of heteroatoms of the heteroaryl group is more preferably 1 or more and 2 or less, and still more preferably 1. In particular, the heteroaryl group is preferably a monovalent group derived from an aromatic heterocyclic ring having 5 or more ring-forming atoms.

Each of the alkyl group, the alkenyl group, the alkynyl group, the aryl group, and the heteroaryl group as the monovalent organic group may be a substituted group. The substituent is not particularly limited, and examples thereof include an alkyl group, an alkoxy group, an alkenyl group, an alkynyl group, a halogen atom, a hydroxy group, a carboxy group, a cyano group, an amino group, a carbamoyl group, and the like. Among them, the substituent is preferably an alkyl group or a halogen atom, and more preferably a methyl group, an ethyl group, or a fluorine atom. The substituent is more preferably a methyl group or a fluorine atom. The substituent does not substitute the same kind of group. For example, a substituent that substitutes an alkyl group does not include an alkyl group.

In addition, in Chemical Formula 1, from the viewpoint of excellent antimicrobial activity, preferably, Y₁ and Y₂ may be bonded to each other to form an optionally substituted nitrogen-containing monocyclic ring containing a 5- or 6-membered ring, and further, R₁ and R₃ may be bonded to each other to form a nitrogen-containing bicyclic ring containing a 5- or 6-membered ring which may be substituted, or two rings constituting the nitrogen-containing bicyclic ring may further be crosslinked to form a nitrogen-containing tricyclic ring containing a 5- or 6-membered ring which may be substituted. That is, in a preferred embodiment of the present aspect, the nitroxyl radical form of the compound of the present aspect is represented by the following Chemical Formula 2.

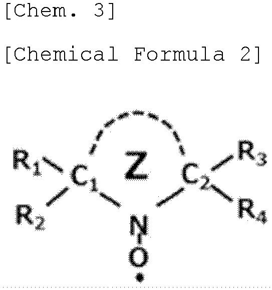

In Chemical Formula 2, R₁ to R₄ are each independently a hydrogen atom or an optionally substituted monovalent organic group, Z is an optionally substituted nitrogen-containing monocyclic ring containing a 5- or 6-membered ring, and in this case, R₁ and R₃ may be bonded to each other to form an optionally substituted nitrogen-containing bicyclic ring containing a 5- or 6-membered ring, or two rings constituting the nitrogen-containing bicyclic ring may be further crosslinked to form an optionally substituted nitrogen-containing tricyclic ring containing a 5- or 6-membered ring.

Examples of the nitrogen-containing monocyclic ring as Z include aliphatic heterocyclic rings such as a pyrrolidine ring, a piperidine ring, a morpholine ring, and a pyrroline ring, and aromatic heterocyclic rings such as a pyrrole ring and an imidazole ring. In addition, examples of the nitrogen-containing bicyclic ring formed by further bonding R₁ and R₃ to each other include a 9-azabicyclo[3.3.1]nonane ring and a 9-aza-3-oxabicyclo[3.3.1]nonane ring. Further, examples of the nitrogen-containing tricyclic ring formed by further crosslinking the two rings constituting the nitrogen-containing bicyclic ring include a 2-azaadamantane ring, a 2,6-diazaadamantane ring, a 6-aza-2-oxaadamantane ring, and a 9-aza-6-noradamantane ring. Among them, from the viewpoint of excellent inactivation activity against microorganisms, the compound of the present aspect preferably has a nitrogen-containing monocyclic ring, a nitrogen-containing bicyclic ring, or a nitrogen-containing tricyclic ring, more preferably has a nitrogen-containing monocyclic ring or a nitrogen-containing tricyclic ring, and particularly preferably has a nitrogen-containing tricyclic ring. In addition, the compound of the present aspect preferably has an aliphatic heterocyclic ring which is a nitrogen-containing monocyclic ring, a 2-azaadamantane ring which is a nitrogen-containing tricyclic ring, or a 9-aza-6-noradamantane ring, and more preferably has a piperidine ring which is a nitrogen-containing monocyclic ring or a 2-azaadamantane ring which is a nitrogen-containing tricyclic ring.

The nitrogen-containing monocyclic ring formed in Chemical Formula 2 or the nitrogen-containing bicyclic ring or the nitrogen-containing tricyclic ring that can be formed in Chemical Formula 2 is an optionally substituted ring structure. The substituent capable of substituting these ring structures is not particularly limited, and examples thereof include an alkyl group, an alkoxy group, an alkenyl group, an alkynyl group, a halogen atom, a hydroxy group, a carboxy group, a carbonyl group, a cyano group, an amino group, a carbamoyl group, a tosyl group, a (halogenated) acylamino group, a phosphate group, and the like. Among them, the substituent is more preferably a methyl group, an ethyl group, a methoxy group, an ethoxy group, a fluorine atom, a hydroxy group, a carboxy group, or a tosyl group.

Here, when the compound of the present aspect does not contain the ring structure or contains a nitrogen-containing monocyclic ring, R₁ to R₄ are each independently preferably a hydrogen atom or an optionally substituted alkyl group, more preferably a hydrogen atom or an unsubstituted alkyl group, further preferably a hydrogen atom or an alkyl group having 1 to 4 carbon atoms, and particularly preferably a hydrogen atom, a methyl group, or an ethyl group. From the viewpoint of antimicrobial activity, when the compound of the present aspect contains the nitrogen-containing bicyclic ring or the nitrogen-containing tricyclic ring, R₂ and R₄ are each independently preferably a hydrogen atom or an optionally substituted alkyl group, more preferably a hydrogen atom or an unsubstituted alkyl group, still more preferably a hydrogen atom or an alkyl group having 1 to 4 carbon atoms, and particularly preferably a hydrogen atom, a methyl group, or an ethyl group.

An example of the compound of the present aspect is as follows (indicated by nitroxyl radical form). For some compounds, the standard redox potential (E⁰@25°C) values between the nitroxyl radical form and the oxoammonium form described above are also shown. In view of the mechanism by which the compound according to the present aspect exhibits excellent antimicrobial activity, it can be said that all of the compounds shown below exhibit high antimicrobial activity.

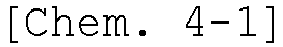

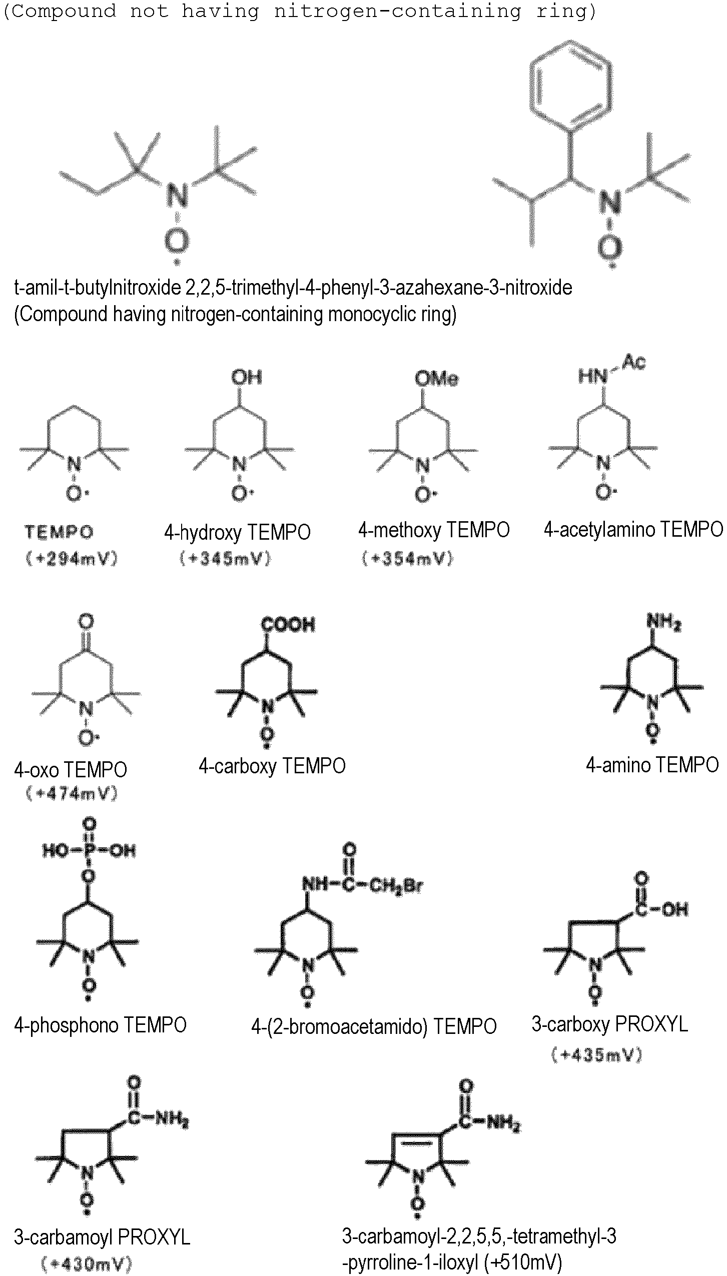

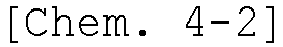

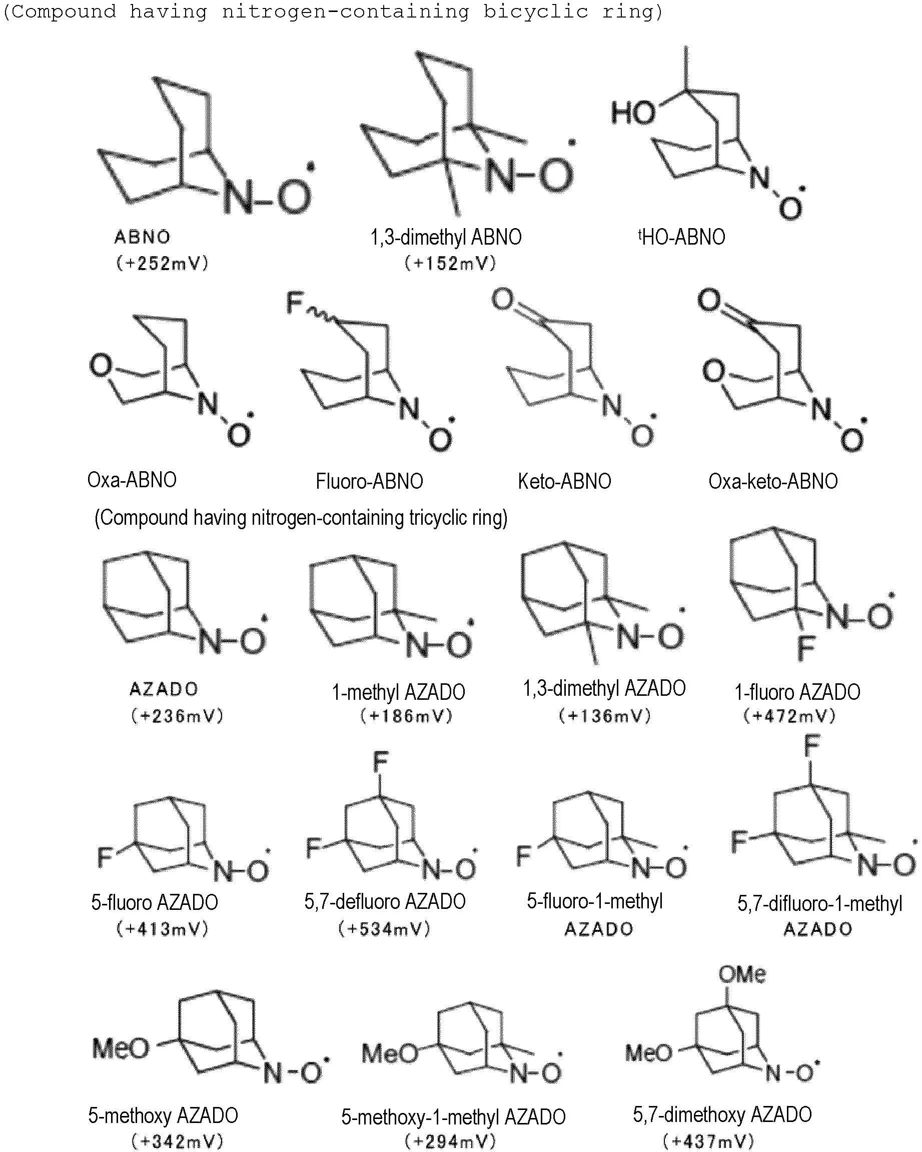

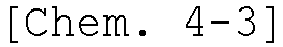

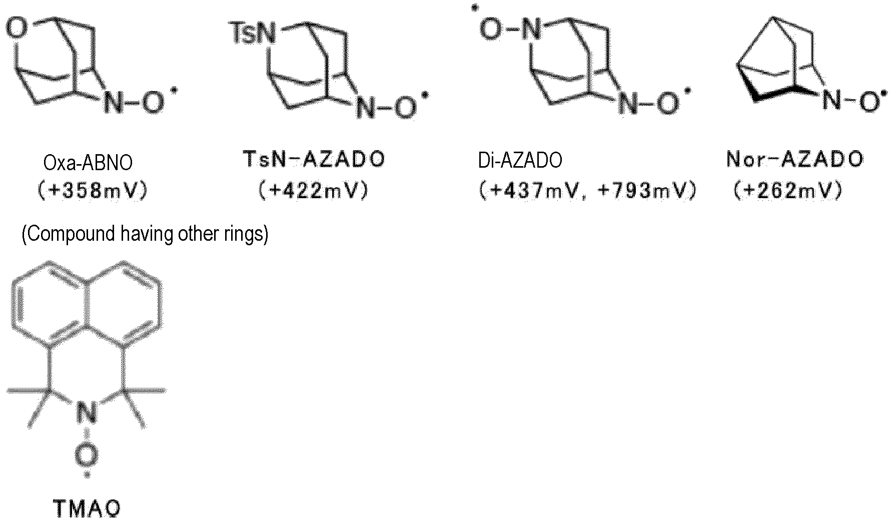

In view of the value of E⁰ described above, when heteroatoms are introduced into a structure of TEMPO, ABNO, AZADO, or the like constituting the skeleton of the compound, the redox characteristics of the compound of the present aspect shift to the high potential side. From this, it is considered that the oxidizing power as the potential of the corresponding oxoammonium form increases.

Among the above compounds, from the viewpoint of antimicrobial activity, the compounds are preferably TEMPO, 4-hydroxy TEMPO, 4-methoxy TEMPO, 4-acetylamino TEMPO, 4-oxoTEMPO, 4-carboxy TEMPO, 4-amino TEMPO, 4-phosphono TEMPO, 4-(2-bromoacetamido) TEMPO, AZADO, 1-methyl AZADO, 1,3-dimethyl AZADO, 1-fluoro AZADO, 5-fluoro AZADO, 5,7-difluoro AZADO, 5-fluoro-1-methyl AZADO, 5,7-difluoro-1-methyl AZADO, 5-methoxy AZADO, 5-methoxy-1-methyl AZADO, 5,7-dimethoxy AZADO, oxa-AZADO, TsN-AZADO, and di-AZADO or Nor-AZADO, more preferably, AZADO, 1-methyl AZADO, 1,3-dimethyl AZADO, 1-fluoro AZADO, 5-fluoro AZADO, 5,7-difluoro AZADO, 5-fluoro-1-methyl AZADO, 5,7-difluoro-1-methyl AZADO, 5-methoxy AZADO, 5-methoxy-1-methyl AZADO, 5,7-dimethoxy AZADO, oxa-AZADO, TsN-AZADO, di-AZADO, Nor-AZADO, TEMPO or 4-acetylamino TEMPO, further preferably AZADO, 1-methyl AZADO, 1,3-dimethyl AZADO, 1-fluoro AZADO, 5-fluoro AZADO, 5,7-difluoro AZADO, 5-fluoro-1-methyl AZADO, 5,7-difluoro-1-methyl AZADO, 5-methoxy AZADO, 5-methoxy-1-methyl AZADO, 5,7-dimethoxy AZADO, TEMPO or 4-acetylamino TEMPO, and particularly preferably 2-azaadamantane-N-oxyl (AZADO), 2,2,6,6-tetramethylpiperidine-N-oxyl (TEMPO) or 4-acetylamino

### TEMPO.

### <Cocatalyst>

From the viewpoint of antimicrobial activity, the inactivation method according to the present aspect preferably brings the compound of the present aspect into contact with a target in the presence of a cocatalyst containing a transition metal. Here, the cocatalyst of the present aspect contains an ion of a transition metal (that is, a salt containing a transition metal) or a simple substance of a transition metal. The transition metal that can be contained in the cocatalyst of the present aspect is not particularly limited as long as the transition metal is a metal element classified into Group 3 elements to Group 12 elements in the periodic table of elements. Among them, from the viewpoint of excellent antimicrobial activity, the transition metal preferably contains one or more selected from the group consisting of Ag, Au, Pt, Pd, Ni, Mn, Fe, Ti, Al, Zn, and Cu, and more preferably contains ions (salts) thereof. In addition, the transition metal further preferably contains Ag or Cu, particularly preferably contains Ag or Cu ions (salts), and most preferably contains Ag ions or divalent Cu ions (Cu(II) ions).

When the cocatalyst contains an ion (salt) of a transition metal, the counteranion of the ion is not particularly limited. Examples of the salt containing a transition metal as a cocatalyst include a sulfate, a sulfite, a hyposulfite, a persulfate, a thiosulfate, a carbonate, a phosphate, pyrophosphoric acid, a hydrochloride, a nitrate, a nitrite, a halide (for example, fluoride, chloride, bromide, or iodide), a trifluoromethanesulfonate, and the like.

When a cocatalyst is present in the inactivation method of the present aspect, the amounts of the compound and the transition metal used are not particularly limited, but from the viewpoint of excellent inactivation activity of microorganisms, the molar ratio of the amounts of these components used is preferably 1 : 2 to 4 : 1 (compound : transition metal), and more preferably 2 : 3 to 3 : 2.

### <Target>

The method for inactivating bacteria or viruses according to the present aspect includes bringing the compound of the present aspect into contact with a target suspected to be contaminated by bacteria or viruses. That is, according to another aspect of the present invention, there is also provided an agent for inactivating bacteria or viruses containing the compound described above. The inactivation of microorganisms using the compound or the inactivating agent of the present aspect can be used by bringing the compound or the inactivating agent into contact with a target suspected to be contaminated by bacteria or viruses, for example, kitchen (kitchen space such as floors and walls of kitchen, and the like), sink, food handling areas such as refrigerators, cooking utensils such as knives and pots, tableware such as plates, refrigerator, toilet, bath, washroom, living spaces such as living rooms and bedrooms, textile products such as sofas, cushions, bedding, and curtains, and the like. Here, bacteria and viruses, which are targets of inactivation, are also not particularly limited, and exhibit an inactivation action on various bacteria, fungi, and viruses. Examples of the bacteria include gram-negative bacteria such as Escherichia species such as Escherichia coli; Pseudomonas species such as Pseudomonas aeruginosa; Salmonella species such as Salmonella choleraesuis; Moraxella species such as Moraxella catarrhalis; and Legionella species such as Legionella pneumophila, gram-positive bacteria such as staphylococcus species such as Staphylococcus aureus; Clostridium species such as Clostridium botulinum and Clostridium perfringens, and the like. Examples of molds include Aspergillus species such as Aspergillus niger and Aspergillus penicilloides; Paecilomyces species such as Paecilomyces variotii; Penicillium species such as Penicillium pinophilum and Penicillium citrinum; Trichoderma species such as Trichoderma virens; Chaetomium species such as Chaetomium globosum; Eurotium species such as Eurotium tonophilum; Rhizopus species such as Rhizopus oryzae; Cladosporium species such as Cladosporium cladosporioides; Aureobasidium species such as Aureobasidium pullulans; Myrothecium species such as Myrothecium verrucaria; and the like. Examples of fungi other than molds include Saccharomyces species such as Saccharomyces cerevisiae, Candida species such as Candida albicans, Rhodotorula species such as Rhodotorula rubra; and the like. Furthermore, the virus may be an enveloped virus or a non-enveloped virus. Examples of the enveloped virus include coronaviruses (including SARS-CoV-2), influenza viruses, herpesviruses, rubella viruses, and the like. Examples of the non-enveloped virus include norovirus, rotavirus, and the like. The target virus is preferably an enveloped virus, more preferably a coronavirus, and particularly preferably a SARS-CoV-2. Among them, from viewpoint of effectively inhibiting the entry of viruses into human cells, the inactivation method according to the present aspect preferably inactivates viruses by inducing oxidative damage to a spike protein constituting the virus. In another preferred embodiment, the target of inactivation is bacteria, and the bacteria is either bacterial or fungal.

The contact is not particularly limited, and examples thereof include a form in which the compound of the present aspect or a composition containing the compound (for example, liquid compositions) is directly sprayed to the target, or a form in which the compound of the present aspect or the composition is filled in a spraying device such as a trigger or an aerosol and sprayed. With such a configuration, the compound of the present aspect can be distributed over a wide range. The contact time between the compound of the present aspect and the target is not limited, and is, for example, preferably 5 seconds or more, more preferably 1 minute or more, and still more preferably 30 minutes or more. The upper limit of the contact time is also not particularly limited, but is preferably 24 hours or less, more preferably 12 hours or less, and still more preferably 6 hours or less. In addition, the form of use when the compound of the present aspect is brought into contact with a target is not particularly limited. For example, the compound can be liquid, gel, or paste. When the above contact is performed in a solution, it may be possible to monitor the progress of the oxidation reaction (for example, denaturation of proteins) by observing the change in color of the solution.

In the case of a liquid form, the compound can be used particularly as a spray, a lotion, or the like. For example, a known spray container such as a trigger spray container (direct pressure or pressure accumulation type) or a dispenser type pump spray container is filled, and the spray amount is adjusted such that the compound can be sprayed to a place where bacteria or viruses are present or suspected to be present.

### <<Antimicrobial or Antiviral Base Material>>

According to still another aspect of the present invention, there is also provided an antimicrobial or antiviral base material, in which a base material is supported or coated with the inactivating agent according to one aspect of the present invention described above and, if necessary, a cocatalyst. By using such an antimicrobial base material, the inactivating agent can be easily used for various applications.

Examples of the base material constituting the antimicrobial base material include a woven fabric, a nonwoven fabric, a filter, a urethane foam, and the like as a material that is inexpensive and can easily support and coat the inactivating agent. The method for supporting or coating the compound and, if necessary, the cocatalyst on these base materials is not particularly limited. Here, as for the base material according to this aspect, as an index of excellent antimicrobial activity, the antiviral activity value (Mv) against influenza virus or feline calicivirus measured based on JIS L 1922:2016 (antiviral test method for textile products) is preferably 2.0 or more, more preferably 2.2 or more, and still more preferably 2.5 or more. Such an antimicrobial base material can be suitably used for applications such as a mask, a medical textile product, an air conditioner, an air cleaner, a refrigerator, a temperature controller, a dehumidifier, a humidifier, and a suction vacuum cleaner filter. It is needless to say that the antimicrobial base material is also used for other applications.

Note that the following embodiments are also included in the scope of the present invention: the inactivation method according to claim 1 having the features of claim 2; the inactivation method according to claim 1 having the features of claim 3; the inactivation method according to any one of claims 1 to 3 having the features of claim 4; the inactivation method according to any one of claims 1 to 4 having the features of claim 5; the inactivation method according to any one of claims 1 to 5 having the features of claim 6; the inactivation method according to claim 5 or 6 having the features of claim 7; the inactivation method according to any one of claims 1 to 7 having the features of claim 8; the inactivation method according to any one of claims 1 to 8 having the features of claim 9; the inactivation method according to any one of claims 1 to 9 having the features of claim 10; the inactivation method according to claim 10 having the features of claim 11; the inactivation method according to any one of claims 1 to 11 having the features of claim 12; the inactivating agent according to claim 13 having the features of claim 14; the base material according to claim 15 using the inactivating agent according to claim 13 or 14.; the base material according to claim 15 having the features of claim 16; the base material according to claim 15 or 16, having the features of claim 17; the base material according to any one of claims 15 to 17 having the features of claim 18; and the base material according to any one of claims 15 to 18 having the features of claim 19.

### [Examples]

Hereinafter, embodiments of the present invention will be described in detail using examples, but the technical scope of the present invention is not limited to only the following examples.

### <<Examination of Dilution Rate of Virus>>

First, for the purpose of examining the influence of feline coronavirus (FCov (VR-989)) on the viability of feline kidney-derived immortalized cells (CRFK), conditions for virus dilution ratios were examined.

Specifically, first, the culture supernatant after culturing CRFK cells infected with FCoV for 3 days was diluted 20 times with PBS to prepare a stock solution of the FCoV solution. In addition, this stock solution was serially diluted 10 times at a time with PBS to 10¹⁰ times to prepare FCoV solutions at respective dilution ratios.

On the other hand, CRFK cells suspended using 10% (v/v) FBS-containing DMEM medium were seeded at 100 µL/well to be 1 × 10⁴ cells/well in a 96 well plate, and cultured for 4 days. After 4 days of culture, the medium was replaced with 100 µL/well of 2% (v/v) FBS-containing DMEM medium, and the FCoV solution at each dilution ratio prepared above was added at 100 µL/well, followed by further culture for 24 hours. Thereafter, the medium was replaced with 200 µL/well of 2% (v/v) FBS-containing DMEM medium, and the cells were cultured for 2 days or 3 days. Then, the cultured cells were evaluated for a cytopathic effect (CPE). For the cytopathic effect (CPE), each well was observed using an optical microscope, and a well in which 50% or more of cells were denatured was evaluated as CPE positive. As a result of the evaluation of CPE, a change in cell viability dependent on the dilution rate was confirmed up to 10⁵ fold dilution in either case after culture for 2 days or 3 days. With reference to this result, the amount of virus to be added was determined in the following experiment.

### <<Confirmation of Antiviral Effect of AZADO (WST-8 Method)>>

For the purpose of confirming whether AZADO, which is one kind of nitroxyl radicals, exhibits an antiviral effect, the following experiment was performed, and the antiviral effect was evaluated by the WST-8 method.

Specifically, first, the culture supernatant after culturing CRFK cells infected with FCoV for 3 days was diluted 20 times with PBS to prepare a stock solution of the FCoV solution. In addition, this stock solution was serially diluted 2 times at a time with PBS to 160 times dilution to prepare FCov solutions at respective dilution ratios. Then, after each serial dilution of the FCoV solution, an oxoammonium form of AZADO (the counteranion is a 100 mM nitrate ion; AZADO-Oxo) or silver nitrate (Comparative example) was added and incubated at room temperature for 2 hours to prepare sample solutions.

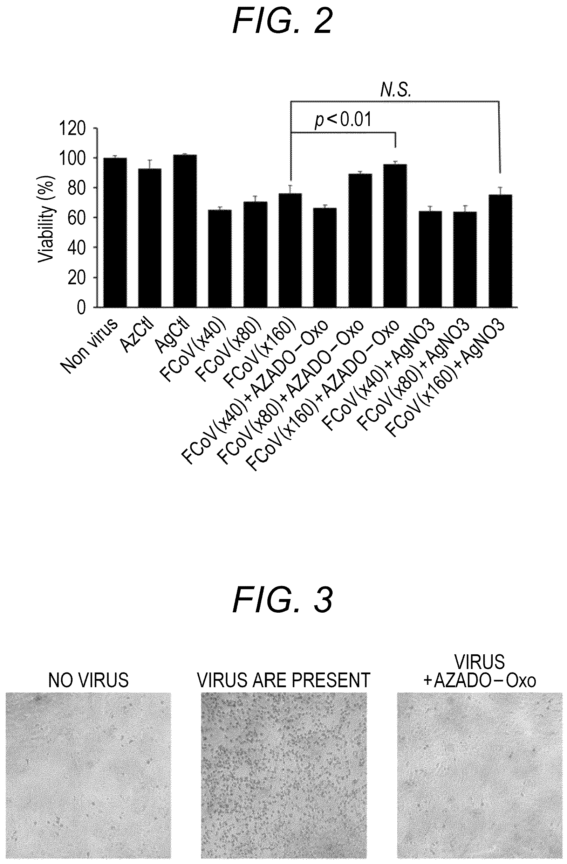

On the other hand, CRFK cells suspended using 10% (v/v) FBS-containing DMEM medium were seeded at 100 µL/well to be 1 × 10⁴ cells/well in a 96 well plate, and cultured for 4 days. After 4 days of culture, the medium was replaced with 100 µL/well of 2% (v/v) FBS-containing DMEM medium, and each sample solution was added at 100 µL/well, followed by culture for 2 days. Thereafter, the cell viability in each sample was measured by the WST-8 method. The WST-8 method is a method for evaluating the presence of living cells by utilizing the fact that a certain tetrazolium salt (WST-8) is reduced by an electron carrier in living cells to generate a highly water-soluble orange formazan dye. The results are shown in Fig. 2. As shown in Fig. 2, by adding AZADO-Oxo, recovery of cell viability decreased by virus addition was observed. On the other hand, in Comparative Example (silver nitrate addition), such a phenomenon was not observed. These results showed that AZADO-Oxo exhibited an excellent antiviral effect.

### <<Confirmation of Antiviral Effect of AZADO (CPE method; Incubation at 37°C))

For the purpose of confirming whether AZADO, which is one kind of nitroxyl radicals, exhibits an antiviral effect, the following experiment was performed, and the antiviral effect was evaluated by the CPE method.

Specifically, first, the culture supernatant after culturing CRFK cells infected with FCoV for 3 days was diluted 20 times with PBS to prepare a stock solution of the FCoV solution. In addition, this stock solution was serially diluted 10 times at a time with PBS to 10⁵ times dilution to prepare FCov solutions at respective dilution ratios. Then, AZADO-Oxo was added to the FCoV solution after each serial dilution to have a final concentration of 2 mM, incubated at 37°C for 2 hours, and further diluted 20 times with PBS to prepare a sample solution. A sample solution of Comparative Example was also prepared without adding AZADO-Oxo.

On the other hand, CRFK cells suspended using 10% (v/v) FBS-containing DMEM medium were seeded at 100 µL/well to be 1 × 10⁴ cells/well in a 96 well plate, and cultured for 4 days. After 4 days of culture, the medium was replaced with 100 µL/well of 2% (v/v) FBS-containing DMEM medium, and each sample solution prepared above was added at 100 µL/well, followed by further culture for 24 hours. Then, the medium was replaced with 200 µL/well of 2% (v/v) FBS-containing DMEM medium and cultured for another 2 days. Then, for the cells after the culture, the cytopathic effect (CPE) was evaluated by the same method as described above. The results are shown in Table 1 below. In addition, "TCID₅₀" described in Table 1 is an abbreviation of 50% tissue culture infectious dose, and is the viral load at which 50% of host cells are killed by viral infection.

**[Table 1]**

| Viral load (relative value) | AZADO-Oxo added | | | No AZADO-Oxo added | | |
|---|---|---|---|---|---|---|
| | Total number of wells | Number of wells when CPE is positive | Positive rate [%] | Total number of wells | Number of wells when CPE is positive | Positive rate [%] |
| 1 | 7 | 0 | 0 | 7 | 7 | 100 |
| 10⁻¹ | 8 | 0 | 0 | 8 | 8 | 100 |
| 10⁻² | 8 | 0 | 0 | 8 | 4 | 50 |
| 10⁻³ | 8 | 0 | 0 | 8 | 2 | 25 |
| 10⁻⁴ | 8 | 0 | 0 | 8 | 0 | 0 |
| 10⁻⁵ | 7 | 0 | 0 | 7 | 0 | 0 |
| TCID₅₀ | 253 | | | 142262 | | |
| logTCID₅₀ | 2.4 | | | 5.2 | | |

As shown in Table 1, it was also confirmed that AZADO-Oxo showed excellent antiviral activity. In addition, the value of ΔlogTCID₅₀, which is an index of antiviral activity, is 2.8 (= 5.2 - 2.4), and this value means that AZADO-Oxo reduced the viral load to 1/10^{2.8} (= about 1/1000). Therefore, it was also confirmed that AZADO-Oxo exhibited sufficient antiviral activity.

### <<Confirmation of Antiviral Effect of AZADO (CPE method; Incubation at 25°C)>>

The antiviral effect was evaluated based on the CPE method in the same manner as described above, except that the temperature at the time of incubation was changed from 37°C to 25°C, and the number of days of culture after the addition of the sample solution and the change of the culture medium was changed from 2 days to 3 days, for the purpose of conforming to the conditions of JIS L 1922:2016 (antiviral test method for textile products). The results are shown in Table 2 below.

**[Table 2]**

| Viral load (relative value) | AZADO-Oxo added | | | No AZADO-Oxo added | | |
|---|---|---|---|---|---|---|
| | Total number of wells | Number of wells when CPE is positive | Positive rate [%] | Total number of wells | Number of wells when CPE is positive | Positive rate [%] |
| 1 | 7 | 0 | 0 | 7 | 7 | 100 |
| 10⁻¹ | 8 | 0 | 0 | 8 | 8 | 100 |
| 10⁻² | 8 | 0 | 0 | 8 | 5 | 62.5 |
| 10⁻³ | 8 | 0 | 0 | 8 | 1 | 12.5 |
| 10⁻⁴ | 8 | 0 | 0 | 8 | 0 | 0 |
| 10⁻⁵ | 7 | 0 | 0 | 7 | 0 | 0 |
| TCID₅₀ | 253 | | | 142262 | | |
| logTCID₅₀ | 2.4 | | | 5.2 | | |

From the results shown in Table 2, it was confirmed that AZADO-Oxo exhibited sufficient antiviral activity even in this experimental setup incubated at 25°C.

In addition, in this experiment, a photograph of the wells observed with a microscope 48 hours after the addition of the sample solution to which AZADO-Oxo was added (virus + AZADO-Oxo) or not added (with virus) is shown in Fig. 3 together with a photograph of cells to which no virus was added (without virus). As shown in Fig. 3, the form of the cell is changed to a round shape by the addition of virus, but this form change is suppressed by the addition of AZADO-Oxo, which also indicates the antiviral effect of AZADO-Oxo.

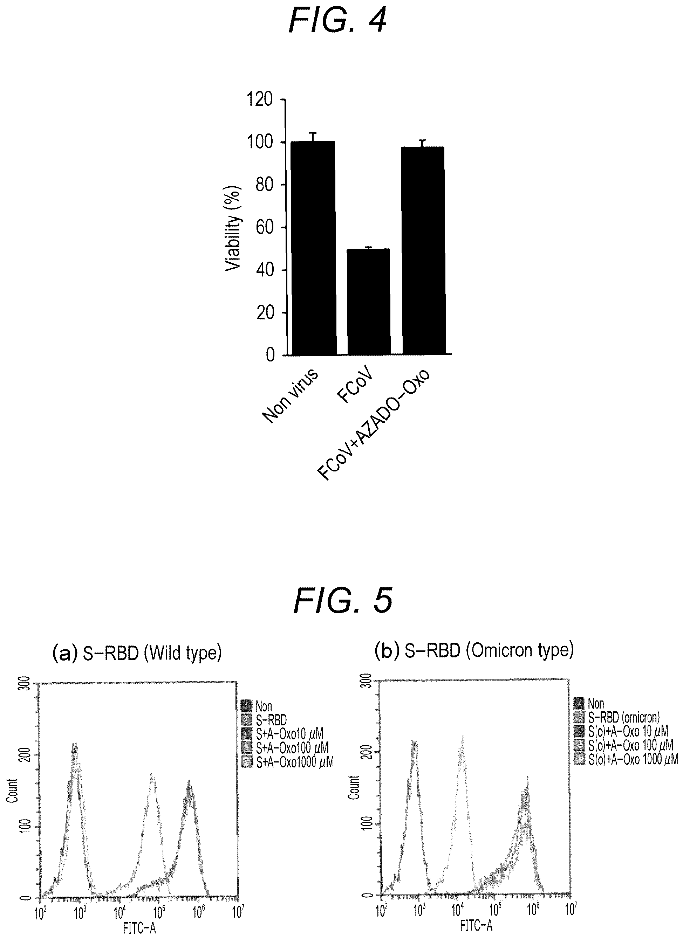

Furthermore, in this experiment, the cell viability was measured using the WST-8 method for cells cultured for 3 days after addition of the sample solution and replacement of the culture medium. The results are shown in Fig. 4. As shown in Fig. 4, the addition of virus reduced the cell viability to about 50% (FCoV), but the addition of AZADO-Oxo (FCoV + AZADO-Oxo) recovered the cell viability to the same level as the blank (Non virus).

### <<Confirmation of Antiviral Effect of AZADO on SARS-Cov-2>>

In this experiment, it was confirmed whether AZADO-Oxo exhibits antiviral activity against currently prevalent SARS-Cov-2. Here, SARS-CoV-2 invades into human cells by binding a spike protein present on the surface of virus particles to ACE2 which is an infection receptor on the surface of human cells. Therefore, it was confirmed whether or not the binding between the spike protein and ACE2 was inhibited by denaturing the spike protein by oxidation by AZADO-Oxo to exhibit antiviral activity.

The virus spike proteins (two types of wild type strain and Omicron strain), cells, and fluorescent-labeled antibodies for detection used in this experiment are as follows:
· Spike protein (wild type strain): SARS-CoV-2 Spike Protein (RBD) (aa319-541), His Tag Recombinant Protein (Thermo, RP-87678) (hereinafter expressed as "S-RBD-His(WT)")
· Spike protein (Omicron strain): SARS-CoV-2 B.1.1.529 (Omicron) Spike RBD Protein (His Tag) (Sino Biological, 40592-V08H121) (hereinafter expressed as "S-RBD-His(Omicron)")
· Cell: ACE2 expression HEK293T (hereinafter expressed as "ACE2-HEK293T")
· Fluorescent-labeled antibody: 6x-His Tag Monoclonal Antibody (AD1.1.10), FITC (Thermo, MA1-81891) (hereinafter expressed as "FITC-anti-6xHis-Tag antibody").

In this experiment, first, 1 µg (2 µL of an aqueous solution having a concentration of 0.5 mg/mL for WT, and 4 µL of an aqueous solution having a concentration of 0.25 mg/mL for Omicron) of each of S-RBD-His(WT) or S-RBD-His(Omicron) was mixed with AZADO-Oxo having a final concentration of 10 µM, 100 µM, or 1000 µM, and subjected to an oxidation treatment at 25°C for 30 minutes to prepare a sample solution. In addition, a sample solution of Comparative Example was also prepared without adding AZADO-Oxo. Next, ACE2-HEK293T was suspended in a microtube containing 100 µL of 1% BSA-containing PBS to be 5 × 10⁵ cells/mL. Thereafter, 4 µL of the sample solution prepared above was added, and the mixture was allowed to stand at 4°C for 30 minutes to perform a binding reaction between the spike protein and the ACE2 receptor on the surface of ACE2-HEK293T cells.

800 µL of 1% BSA-containing PBS was added to the cell suspension after the above reaction, and centrifuged at 400× g for 4 minutes at 4°C. After discarding the supernatant, 100 µL of 1% BSA-containing PBS was added and resuspended. Then, 1 µg (10 µL) of FITC-anti-6xHis-Tag antibody was added, and the mixture was allowed to stand at 4°C for 30 minutes under light-shielding conditions. Thereafter, 1 mL of 1% BSA-containing PBS was further added, and the mixture was centrifuged at 400× g for 4 minutes at 4°C. After discarding the supernatant, 300 µL of 1% BSA-containing PBS was added and resuspended. The solution obtained in this manner was evaluated by flow cytometry. When this experiment was performed using HEK293T not expressing ACE2, the binding amount of S-RBD-His was markedly reduced, and thus it was determined that ACE2-specific binding could be detected. The results for the wild type strain (S-RBD-His (WT)) are shown in (a) of Fig. 5, and the results for the Omicron strain (S-RBD-His(Omicron)) are shown in (b) of Fig. 5. Here, as the binding amount between the spike protein and ACE2 decreases (that is, as the binding of these compounds is inhibited by AZADO-Oxo), the peak shifts to the side with a smaller fluorescence intensity (left side in the drawing) .

From the results shown in Fig. 5, when AZADO-Oxo was not present (S), the peak was significantly shifted to the right (indicating that the binding amount to ACE2 was large). In addition, it was confirmed that the peak shifted to the right was shifted to the left depending on the amount of AZADO-Oxo added (the larger the amount added, the greater the shift to the left). Incidentally, the shift of the peak to the left by the addition of AZADO-Oxo was gentle in the Omicron strain as compared with the wild type strain, but it was determined that a certain inactivation effect was obtained also for the Omicron strain.

### <<Comparison of Antiviral Effects between Compounds on SARS-Cov-2>>

For the purpose of comparing the antiviral effect between the compounds, using the oxoammonium form of AZADO (the counteranion is a BF₄⁻ ion; AZADO⁺BF₄⁻), the oxoammonium form of TEMPO (the counteranion is BF₄⁻ ion; TEMPO⁺BF₄⁻) and the oxoammonium form of 4-acetylamino TEMPO (the counteranion is BF₄⁻ ion; 4-AcNH-TEMPO⁺BF₄⁻), the antiviral effect was confirmed by the same method as described above.

Specifically, first, 1 µg of S-RBD-His(WT) (2 µL of an aqueous solution having a concentration of 0.5 mg/mL) was mixed with AZADO⁺BF₄⁻, TEMPO⁺BF₄⁻, or 4-AcNH-TEMPO⁺BF₄⁻ having a final concentration of 10µM, 100µM, or 1000uM, and subjected to an oxidation treatment at 25°C for 30 minutes to prepare a sample solution. In addition, a sample solution of Comparative Example was also prepared without adding the above compound. Next, ACE2-HEK293T was suspended in a microtube containing 100 µL of 1% BSA-containing PBS (containing 2 mM EDTA) to be 1 × 10⁶ cells/mL. Thereafter, 4 µL of the sample solution prepared above was added, and the mixture was allowed to stand at 4°C for 30 minutes to perform a binding reaction between the spike protein and the ACE2 receptor on the surface of ACE2-HEK293T cells.

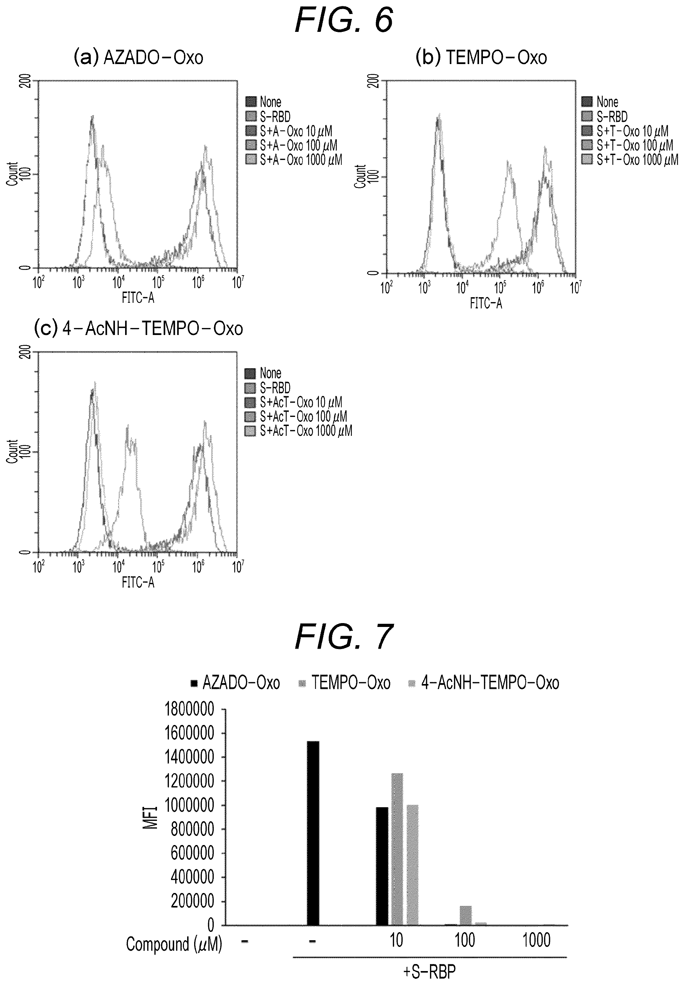

800 µL of 1% BSA-containing PBS (containing 2 mM EDTA) was added to the cell suspension after the above reaction, and the mixture was centrifuged at 400× g for 4 minutes at 4°C. The supernatant was discarded, and then 100 µL of 1% BSA-containing PBS (containing 2 mM EDTA) was added and resuspended. Then, 1 µg (10 µL) of FITC-anti-6xHis-Tag antibody was added, and the mixture was allowed to stand at 4°C for 30 minutes under light-shielding conditions. Thereafter, 1 mL of 1% BSA-containing PBS (containing 2 mM EDTA) was further added, and the mixture was centrifuged at 400× g for 4 minutes at 4°C. The supernatant was discarded, and then 300 µL of 1% BSA-containing PBS (containing 2 mM EDTA) was added and resuspended. The solution obtained in this manner was evaluated by flow cytometry. The results for AZADO⁺BF₄⁻ (AZADO-Oxo) are shown in (a) of Fig. 6 (S represents spike protein, A represents compound), the results for TEMPO⁺BF₄⁻ (TEMPO-Oxo) are shown in (b) of Fig. 6 (S represents spike protein, T represents compound), and the results for 4-AcNH-TEMPO⁺BF₄⁻ (Ac-TEMPO-Oxo) are shown in (c) of Fig. 6 (S represents spike protein, AcT represents compound). In addition, Fig. 7 is a graph showing the results of graphing a mean value of fluorescence intensity (mean fluorescence intensity) at each peak shown in Fig. 6.

From the results shown in Fig. 6, when AZADO-Oxo was not present (S), the peak was largely shifted to the right. The peak shifted to the right was shifted to the left depending on the amount of the compound added. When the activity among the compounds was compared, the activities of AZADO⁺BF₄⁻ and 4-AcNH-TEMPO⁺BF₄⁻ were almost equally strong, and the activity of TEMPO⁺BF₄⁻ was slightly weaker. In addition, the MFI graph of Fig. 7 shows the similar result.

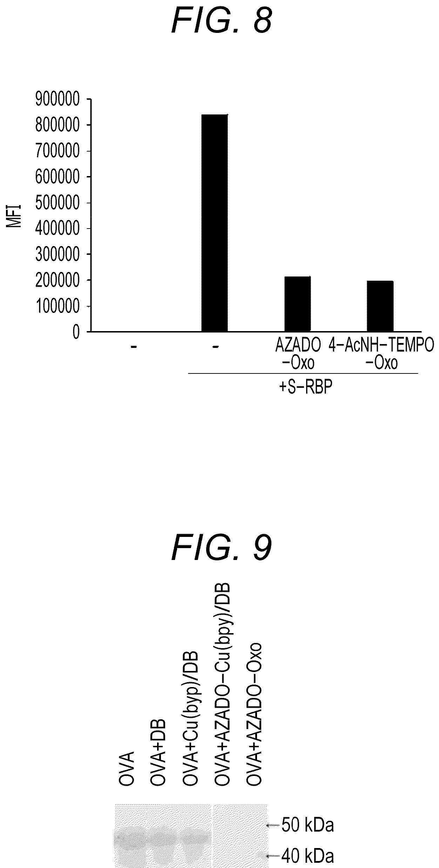

Furthermore, the same operation was performed for AZADO⁺BF₄⁻ and 4-AcNH-TEMPO⁺BF₄⁻ (final concentration 10 µM)) which showed high antiviral effects in the above experiments, but the treatment time when added to S-RBD-His(WT) was extended from 30 minutes to 6 hours. The MFI graph obtained in this manner is shown in Fig. 8. As shown in Fig. 8, by extending the treatment time, AZADO⁺BF₄⁻ and 4-AcNH-TEMPO⁺BF₄⁻ showed higher antiviral activity.

From the results shown above, according to the present invention, there is provided inexpensive means capable of exhibiting an inactivation effect on microorganisms without assuming the presence of light while minimizing the burden on the environment.

### <<Evaluation of Degradation Activity of AZADO on Ovalbumin (OVA)>> (Preparation Example of Catalyst Support (AZADO/DB))

A catalyst support in which AZADO was supported on a catalyst support (carbon support) was prepared by the following method.

5 mg (0.13 mmol) of AZADO and 100 mL of dichloromethane were added to a 300 mL eggplant-shaped flask and the AZADO was dissolved using ultrasound. 100 mg of Denka Black (registered trademark) (DB), which is a carbon support, was added and dispersed using ultrasound for 30 minutes, and then the mixture was stirred at room temperature for 30 minutes. Thereafter, the mixture was evaporated to dryness at 40°C and 600 hPa using a rotary evaporator, and further dried under vacuum for 10 hours to obtain a catalyst support (AZADO/DB).

### (Preparation Example of Catalyst Support (AZADO-Cu(bpy)/DB))

0.03 mmol (10.9 mg) of Cu(OTf)₂, 0.03 mmol (4.7 mg) of bpy, and 0.03 mmol (4.6 mg) of AZADO were each added to a 5 mL vial, dissolved in 3 mL of acetonitrile, and added to a 300 mL eggplant-shaped flask containing 90 mL of acetonitrile. After each reagent was added, the vial wall was washed with acetonitrile and added to the flask. Subsequently, 100 mg of Denka Black (DB) as a carbon support was added to the flask and dispersed with ultrasonic waves for 30 minutes, and then the mixture was stirred at room temperature for 30 minutes. Thereafter, the mixture was evaporated to dryness at 60°C and 250 hPa using a rotary evaporator and further dried under vacuum for 10 hours to obtain a catalyst support (AZADO-Cu(bpy)/DB) on which a cocatalyst was supported together with AZADO. Incidentally, a catalyst support (Cu(bpy)/DB) in which only the cocatalyst was supported was also prepared by performing the same method without using AZADO.

### (Treatment of Ovalbumin (OVA) by Catalyst Support (1))

1 mg each of the AZADO/DB, AZADO-Cu(bpy)/DB, Cu(bpy)/DB, and Denka Black (DB) prepared above was weighed in a microtube, and mixed with 20 µL of a 10 mg/mL ovalbumin (OVA) aqueous solution and 980 µL of pure water, respectively, to prepare a sample. In addition, a sample was similarly prepared using 3 µL of AZADO-Oxo aqueous solution (concentration 100 mM) in place of the catalyst support (the amount of pure water was 977 µL). Further, only OVA and pure water were mixed to prepare a control sample.

Each sample was allowed to stand at room temperature for 24 hours, and then centrifuged at 15000× g for 3 minutes at room temperature. Then, 80 µL of the supernatant of each sample was transferred to another tube, and 20 µL of 5× sample buffer (SB) was added. Thereafter, the plate was allowed to stand at 95°C for 5 minutes to perform electrophoresis together with a molecular weight marker, thereby performing CBB staining. The results are shown in Fig. 9. As shown in Fig. 9, in the sample treated with AZADO-Oxo ("OVA+AZADO-Oxo" in Fig. 9), the band of OVA was almost eliminated. This confirms that the antiviral activity of AZADO-Oxo confirmed above is due to degradation by acting on and oxidizing viral spike proteins. In addition, in the sample in which AZADO was supported on the catalyst support together with the cocatalyst and OVA was treated ("OVA+AZADO-Cu(bpy)/DB" in Fig. 9), the band of OVA was completely eliminated, and the OVA degradation activity was superior to that of AZADO-Oxo. This is considered to be a result of showing that OVA is more strongly oxidized by generating a catalytic cycle of redox reaction by the cooperative action of AZADO and the cocatalyst. In Fig. 9, in the sample to which the carbon support (DB) was added (OVA+DB) and the sample to which Cu(bpy)/DB was added (OVA+Cu(bpy)/DB), the band of OVA was hardly eliminated, and a band equivalent to that of the control (OVA) was confirmed.

### (Treatment of Ovalbumin (OVA) by Catalyst Support (2))

Each sample was prepared by the same method as described above. However, the amount of AZADO-Oxo aqueous solution (concentration 100 mM) added was 2.5 µL.

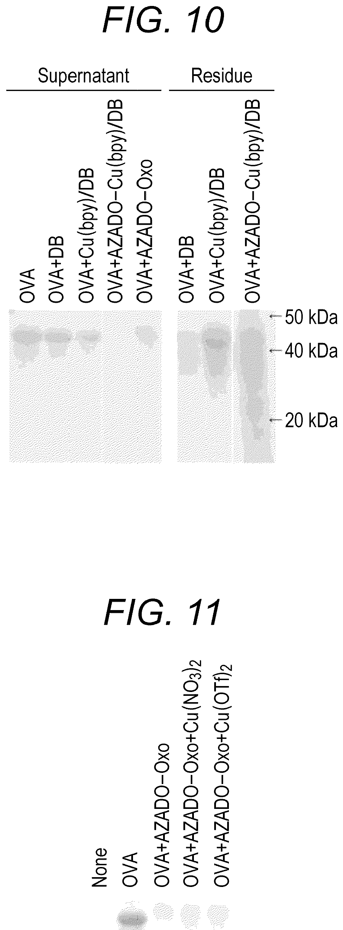

Each sample was allowed to stand at room temperature for 18 hours, and then centrifuged at 15000× g for 3 minutes at room temperature. Then, 80 µL of the supernatant of each sample was transferred to another tube, 20 µL of 5×SB was added, and then the mixture was allowed to stand at 95°C for 5 minutes to prepare a supernatant sample. In addition, for the sample containing the catalyst support (C), the supernatant remaining after the centrifugation was discarded, washed twice with 1 mL of pure water, and resuspended in 80 µL of pure water. Then, 20 µL of 5×SB was added, and then the mixture was allowed to stand at 95°C for 5 minutes to prepare a residue sample. The supernatant sample and the residue sample obtained in this manner were subjected to electrophoresis together with a molecular weight marker, and CBB staining was performed. The results are shown in Fig. 10. As shown in Fig. 10, the same results as in Fig. 9 were confirmed for the supernatant sample. On the other hand, an extremely strong band of OVA was confirmed in the sample to which Cu(bpy)/DB was added ("OVA+Cu(bpy)/DB" in Fig. 10) among the residue samples. From this, it is considered that Cu(bpy)/DB strongly adsorbed OVA. In addition, in the sample in which AZADO was supported on the catalyst support together with the cocatalyst and OVA was treated ("OVA+AZADO-Cu(bpy)/DB" in Fig. 10), it was confirmed that the band of OVA to which Cu(bpy)/DB was adsorbed was remarkably smeared (broadened). From this, it was considered that Cu(bpy)/DB adsorbed OVA to induce oxidative degradation by AZADO as a mechanism in which AZADO-Cu(bpy)/DB eliminates the band of OVA (degradation of OVA) in Figs. 9 and 10 (supernatant sample). According to this mechanism, it is considered that the method and agent for inactivating microorganisms according to one aspect of the present invention exhibit high inactivation activity not only against viruses but also against bacteria, mold, fungus, and the like having proteins as an essential constituent.

### (Treatment of Ovalbumin (OVA) with Unsupported AZADO)

The following samples were prepared respectively:
(a1) A sample of 1000 µL of pure water (None)
(a2) A sample obtained by mixing 980 µL of pure water with 20 µL of a 10 mg/mL ovalbumin aqueous solution (OVA)
(a3) A sample obtained by mixing 980 µL of pure water with 20 µL of a 10 mg/mL ovalbumin aqueous solution and 2.5 µL of a 100 mM AZADO-Oxo aqueous solution (OVA+AZADO-Oxo)
(a4) A sample obtained by mixing 980 µL of pure water with 20 µL of a 10 mg/mL ovalbumin aqueous solution, 2.5 µL of a 100 mM AZADO-Oxo aqueous solution, and 2.5 µL of a 100 mM Cu(NO₃)₂ aqueous solution (OVA+AZADO-Oxo+Cu(NO₃)₂)
(a5) A sample obtained by mixing 980 µL of pure water with 20 µL of a 10 mg/mL ovalbumin aqueous solution, 2.5 µL of a 100 mM AZADO-Oxo aqueous solution, and 2.5 µL of a 100 mM Cu(OTf)₂ aqueous solution (OVA+AZADO-Oxo+Cu(OTf)₂)

Each sample was allowed to stand at room temperature for 24 hours, and then centrifuged at 15000× g for 3 minutes at room temperature. Then, 80 µL of the supernatant of each sample was transferred to another tube, and 20 µL of 5×SB was added. Thereafter, the plate was allowed to stand at 95°C for 5 minutes to perform electrophoresis together with a molecular weight marker, thereby performing CBB staining. The results are shown in Fig. 11. As shown in Fig. 11, in the sample in which AZADO-Oxo was added to OVA, elimination of a band of OVA (protein degradation activity) was confirmed in the same manner as described above, but this effect was not enhanced by the addition of the cocatalyst (Cu(NO₃)₂ or Cu(OTf)₂).

### <<Evaluation of Inactivation Activity against Bacteria and Fungi (Mold)>>

For the oxoammonium form of TEMPO (the counteranion: NO₃⁻ ion; TEMPO⁺NO₃⁻), the oxoammonium form of AZADO (the counteranion is NO₃⁻ ion; AZADO⁺NO₃⁻), and the oxoammonium form of 4-acetylamino TEMPO (the counteranion is NO₃⁻ ion; and 4-AcNH-TEMPO⁺NO₃⁻), which are the compounds according to the invention, the inactivation activity against bacteria and fungi (mold) was evaluated. In Comparative Example, copper(II) nitrate was used.

Specifically, first, Escherichia coli (Escherichia coli ATCC 43895, serotype O157:H7, verotoxin type I and II producing strain), Staphylococcus aureus subsp. aureus NBRC 12732, and Penicillium citrinum (NBRC 6352) were prepared as inactivation targets. The preculture of these targets was performed for Escherichia coli and Staphylococcus aureus in a normal agar medium (produced by Eiken Chemical Co., Ltd.) at 35°C ± 1°C for 18 to 24 hours. In addition, for Penicillium citrinum, the preculture was performed on Potato Dextrose Agar (produced by Difco) at 25°C ± 1°C for 7 to 10 days. Then, a bacterial suspension was prepared using purified water for Escherichia coli, using physiological saline for Staphylococcus aureus, and using a 0.005% sodium dioctyl sulfosuccinate solution for Penicillium citrinum.

Then, to 5 mL of the aqueous solution containing each of the above compounds, 0.05 mL of the bacterial suspension adjusted to have the number of bacteria of 1 × 10⁷ to 1 × 10⁸/mL was added, and the resulting mixture was gently stirred. Here, the concentration of the compound in the aqueous solution was 10 mM or 20 mM in the test of Escherichia coli, 0.1 mM or 1 mM in the test of Staphylococcus aureus, and 0.1 mM or 10 mM in the test of Penicillium citrinum.

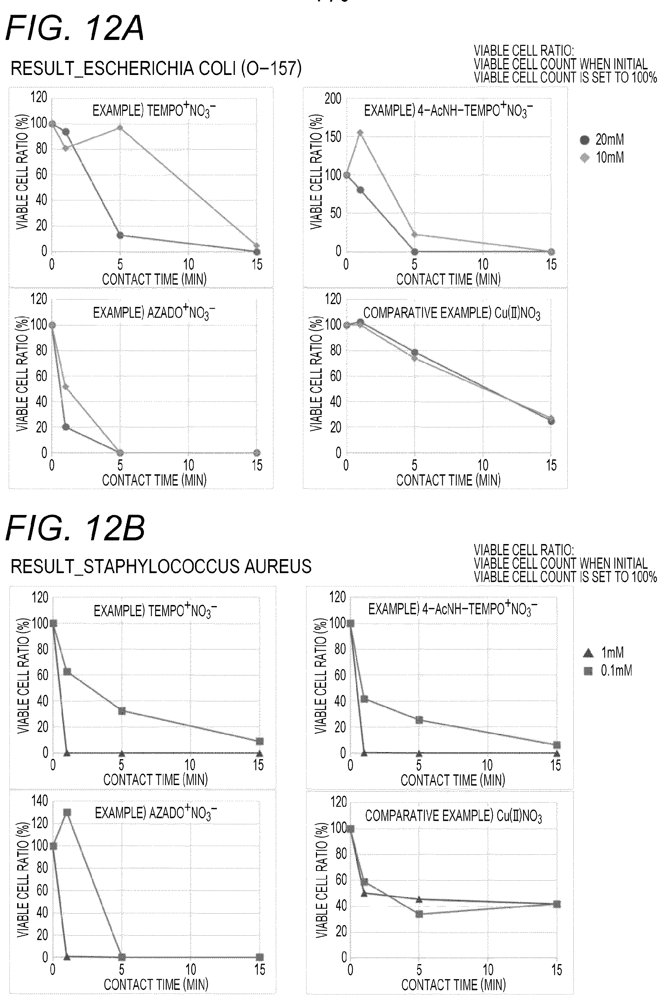

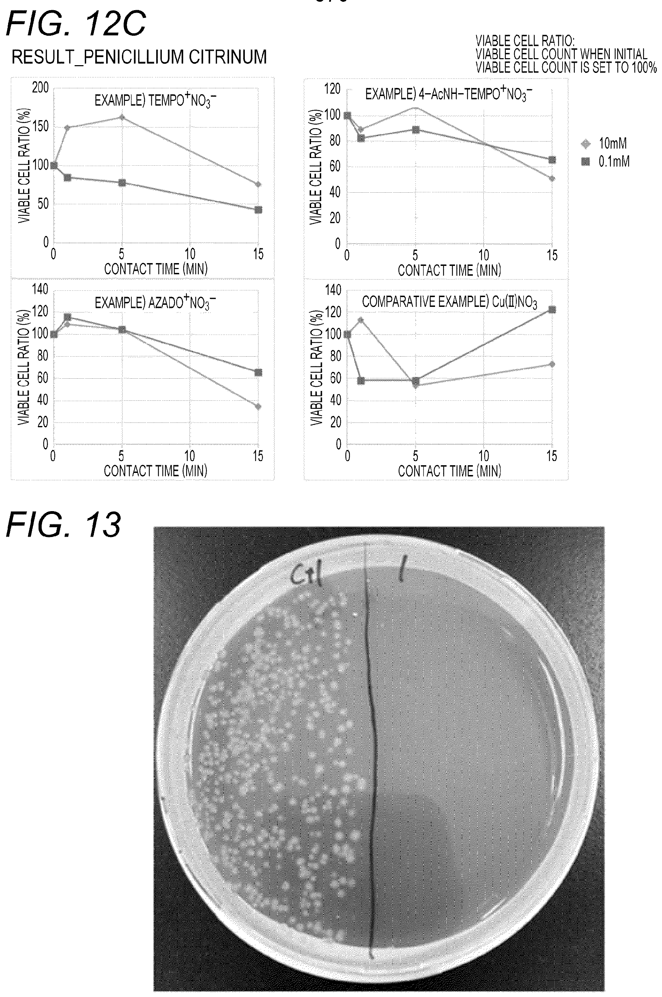

After 1 minute, 5 minutes, and 15 minutes from the addition of the bacterial suspension, the solution was sampled to such an extent that the number of grown colonies could be counted, and diluted with an SCDLP medium (produced by SHIOTANI M.S. CO.,LTD) to neutralize the compound. Thereafter, the neutralized sample was applied to an SCDLP agar medium (produced by SHIOTANI M.S. CO.,LTD) for Escherichia coli and Staphylococcus aureus, and a GPLP agar medium (produced by SHIOTANI M.S. CO.,LTD) for Penicillium citrinum, and subjected to plate culture at 35°C ± 1°C for Escherichia coli and Staphylococcus aureus and at 25°C ± 1°C for Penicillium citrinum by a pour plate culture method, the viable cell count on the medium was counted, and the viable cell count ratio [%] when the viable cell count in Comparative Example was set as 100% was calculated as the viable cell ratio. In addition, the period of the plate culture was set to 2 days for Escherichia coli and Staphylococcus aureus, and 7 days for the Penicillium citrinum. The results of graphing the viable cell ratio calculated for each compound in this manner are shown in Fig. 12A for Escherichia coli, shown in Fig. 12B for Staphylococcus aureus, and shown in Fig. 12C for Penicillium citrinum.

For Escherichia coli, as shown in Fig. 12A, in the sample to which the compound according to the present invention was added, the viable cell ratio was almost 0% 15 minutes after the addition, but the viable cell ratio did not decrease to 0% in Comparative Example (copper(II) nitrate). For Staphylococcus aureus, as shown in Fig. 12B, the inactivation effect of the compound according to the present invention was high, and the viable cell ratio decreased immediately after addition even at a very low concentration. On the other hand, in Comparative Example (copper(II) nitrate)), the viable cell ratio did not decrease after 5 minutes from the addition. For Penicillium citrinum, as shown in Fig. 12C, in the sample to which the compound according to the present invention was added, a trend of decreasing viable cell ratio was observed as time passed after addition, but in Comparative Example (copper(II) nitrate), the viable cell ratio started to increase after 5 minutes from the addition.

### <<Measurement of Minimum Inhibitory Concentration (MIC) against Bacteria (Escherichia Coli)>>

For the oxoammonium form of AZADO (the counteranion: NO₃⁻ ion; AZADO⁺NO₃⁻) which is the compound according to the invention, the minimum inhibitory concentration (MIC) against Escherichia coli was measured by the following method.

First, Escherichia coli (DH5α strain; AMP+) was cultured with shaking in LB medium for 20 hours, and the obtained culture solution was used as an Escherichia coli stock solution. Then, the Escherichia coli stock solution was diluted 1000 times with PBS to obtain a diluted solution. In addition, this diluted solution was used as a comparative example.

On the other hand, AZADO⁺NO₃⁻ was added to the diluted solution obtained above to have a final concentration of 1 µM, 10 µM, 100 µM, 1 mM, or 10 mM, respectively, and the obtained mixture was allowed to stand at room temperature for 15 minutes. Thereafter, for the purpose of deactivating AZADO⁺NO₃⁻, the diluted solution was further diluted 10 times using LB medium as a reaction stop solution. Finally, 25 µL of the reaction solution obtained in this manner was applied to LB agar medium, and cultured at 37°C for 1 day. Then, the number of colonies on the agar medium after culture was counted, and the colony forming unit (CFU/mL) per mL was calculated. The results are shown in Table 3 below. In addition, the "CFU relative value" described in Table 3 is a relative value when the value of the colony forming unit of Comparative Example is 100. Further, Fig. 13 shows a photograph of the agar medium after culture using a sample having a final concentration of AZADO⁺NO₃⁻ of 1 mM.

**[Table 3]**

| AZADO⁺NO₃⁻ final concentration [mM] | Number of colonies | Escherichia coli dilution ratio | LB medium dilution ratio | Application volume [mL] | CFU/mL | CFU relative value |
|---|---|---|---|---|---|---|
| 0 | 303 | 1000 | 10 | 0.025 | 1.21 × 10⁸ | 100 |
| 0.001 | 319 | 1000 | 10 | 0.025 | 1.28 × 10⁸ | 105 |
| 0.01 | 313 | 1000 | 10 | 0.025 | 1.25 × 10⁸ | 103 |
| 0.1 | 151 | 1000 | 10 | 0.025 | 6.04 × 10⁷ | 50 |
| 1 | 0 | 1000 | 10 | 0.025 | 0 | 0 |
| 10 | 0 | 1000 | 10 | 0.025 | 0 | 0 |

From the results shown in Table 3, a remarkable inactivation effect was observed when the final concentration of AZADO⁺NO₃⁻ was 100 µM or more. In addition, a high sterilization effect of 99% or more was confirmed when the final concentration of AZADO⁺NO₃⁻ was 1 mM or more. Note that this concentration of 1 mM is about the same as the concentration at which it was confirmed that a remarkable antiviral action was exhibited in the FCoV infection experiment on CRFK cells. Therefore, it was suggested that the concentration of the disinfecting action of the compound according to the present invention against Escherichia coli was comparable to that of the compound having an antiviral action.

### <<Evaluation of Degradability against Proteins in Mist State>>

In order to simulate a state where a nitroxyl radical exhibits an antiviral action or a disinfecting action on a filter that is present in a living environment, the antiviral action of a carbon material (AZADO-Cu(bpy)/DB) that supports a nitroxyl radical (AZADO) and a cocatalyst (Cu) was evaluated by the following method using a sprayer, using the degradation action of S-RBD as an index.

### (Confirmation of Reactivity in Liquid Phase System)

First, 100 pmol of S-RBD and 0.1 mg of AZADO-Cu(bpy)/DB were mixed in 110 µL of an aqueous solvent (S-RBD : Cu : AZADO = 1 : 250 : 250 (molar ratio)). Then, the mixture was incubated at room temperature for 2 or 6 hours. In addition, an experiment using Cu(bpy)/DB that does not support AZADO was also performed as a comparative example.

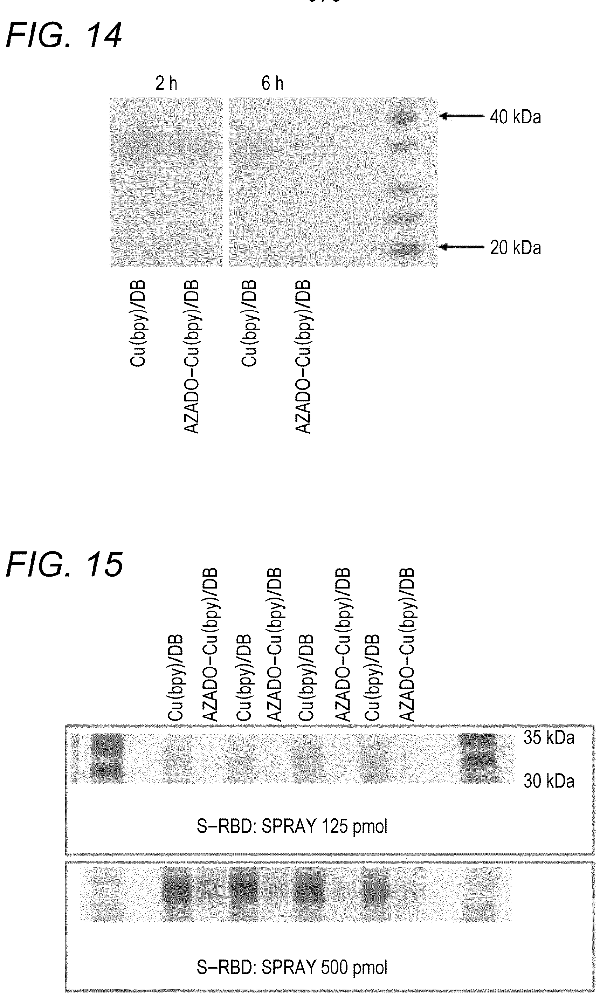

Thereafter, 1×SB was added to the residue obtained by centrifugation, proteins in the residue were sampled, and the amount of S-RBD contained in the residue was evaluated by the SDS-PAGE method and the CBB staining method. The results (electrophoretic photographs) are shown in Fig. 14.

From the results shown in Fig. 14, it was confirmed that the band of S-RBD was eliminated depending on the incubation time in the system to which AZADO-Cu(bpy)/DB was added. Based on this result, the following experiment using a sprayer was performed.

### (Confirmation of Reactivity against Proteins in Mist State)

100 µL of an aqueous solution containing 0.1 mg of AZADO-Cu(bpy)/DB was permeated into a membrane filter (8 mm punch) to produce an antiviral filter. This filter was installed in the opening of a 200 µL microchip and fixed using parafilm. In addition, a filter of Comparative Example was also produced using Cu(bpy)/DB.

For the filter produced above, 25 µL of an aqueous solution containing S-RBD in an amount of 125 pmol or 250 pmol was sprayed from the tip of the microchip using a micro sprayer (Micro FPS, produced by Toray Precision Co., Ltd.), and incubated at room temperature for 24 hours. Thereafter, the amount of S-RBD remaining on the filter was evaluated by the SDS-PAGE method and the silver staining method. The results (electrophoretic photographs) are shown in Fig. 15.

From the results shown in Fig. 15, it was confirmed that the band of S-RBD was eliminated or decreased in the filter containing AZADO-Cu(bpy)/DB regardless of the amount of sprayed S-RBD.

The present application is based on Japanese Patent Application No. 2022-136098 filed on August 29, 2022, the disclosure content of which is incorporated herein by reference in its entirety.

## Claims

1. A method for inactivating bacteria or viruses, comprising bringing a compound having a redox mechanism below into contact with a target suspected to be contaminated by bacteria or viruses: 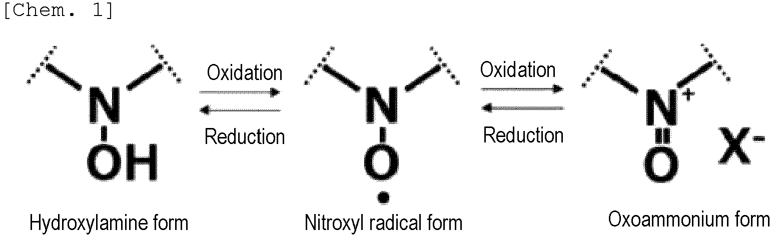 in the formula, X⁻ represents a counteranion, and a broken line represents a bonding position with another atom.

2. The method for inactivating bacteria or viruses according to claim 1, wherein the virus is inactivated by inducing oxidative damage to a spike protein constituting the virus.

3. The method for inactivating bacteria or viruses according to claim 1, wherein the method is a method for inactivating bacteria, and the bacteria are either bacterial or fungal.

4. The method for inactivating bacteria or viruses according to claim 1 or 2, wherein a standard redox potential (25°C) between the nitroxyl radical form and the oxoammonium form is +100 mV to +1000 mV [Ag/Ag⁺].

5. The method for inactivating bacteria or viruses according to claim 1, wherein the nitroxyl radical form of the compound is represented by Chemical Formula 1 below: 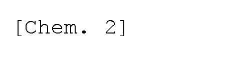 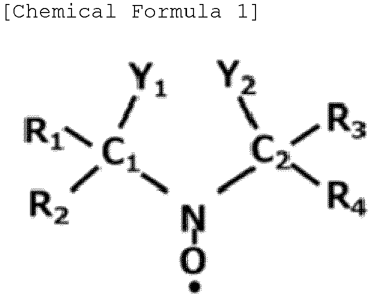 in the formula, R₁ to R₄, Y₁, and Y₂ are each independently a hydrogen atom or an optionally substituted monovalent organic group, and in this case, Y₁ and Y₂ may be bonded to each other to form an optionally substituted nitrogen-containing monocyclic ring containing a 5- or 6-membered ring, and further, R₁ and R₃ may be bonded to each other to form an optionally substituted nitrogen-containing bicyclic ring containing a 5- or 6-membered ring, or two rings constituting the nitrogen-containing bicyclic ring may be further crosslinked to form an optionally substituted nitrogen-containing tricyclic ring containing a 5- or 6-membered ring.

6. The method for inactivating bacteria or viruses according to claim 5, wherein the nitroxyl radical form of the compound is represented by Chemical Formula 2 below: 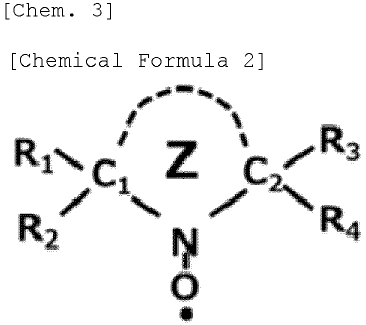 in the formula, R₁ to R₄ are each independently a hydrogen atom or an optionally substituted monovalent organic group, Z is an optionally substituted nitrogen-containing monocyclic ring containing a 5- or 6-membered ring, and in this case, R₁ and R₃ may be bonded to each other to form an optionally substituted nitrogen-containing bicyclic ring containing a 5- or 6-membered ring, or two rings constituting the nitrogen-containing bicyclic ring may be further crosslinked to form an optionally substituted nitrogen-containing tricyclic ring containing a 5- or 6-membered ring.

7. The method for inactivating bacteria or viruses according to claim 5 or 6, wherein
when the compound does not contain the monocyclic ring, the bicyclic ring, or the tricyclic ring, or contains the nitrogen-containing monocyclic ring, R₁ to R₄ are each independently a hydrogen atom or an alkyl group having 1 to 4 carbon atoms, and
when the compound contains the nitrogen-containing bicyclic ring or the nitrogen-containing tricyclic ring, R₂ and R₄ are each independently a hydrogen atom or an alkyl group having 1 to 4 carbon atoms.

8. The method for inactivating bacteria or viruses according to claim 1 or 2, wherein
the nitroxyl radical form of the compound is one or more selected from a group below:

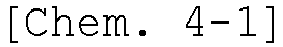

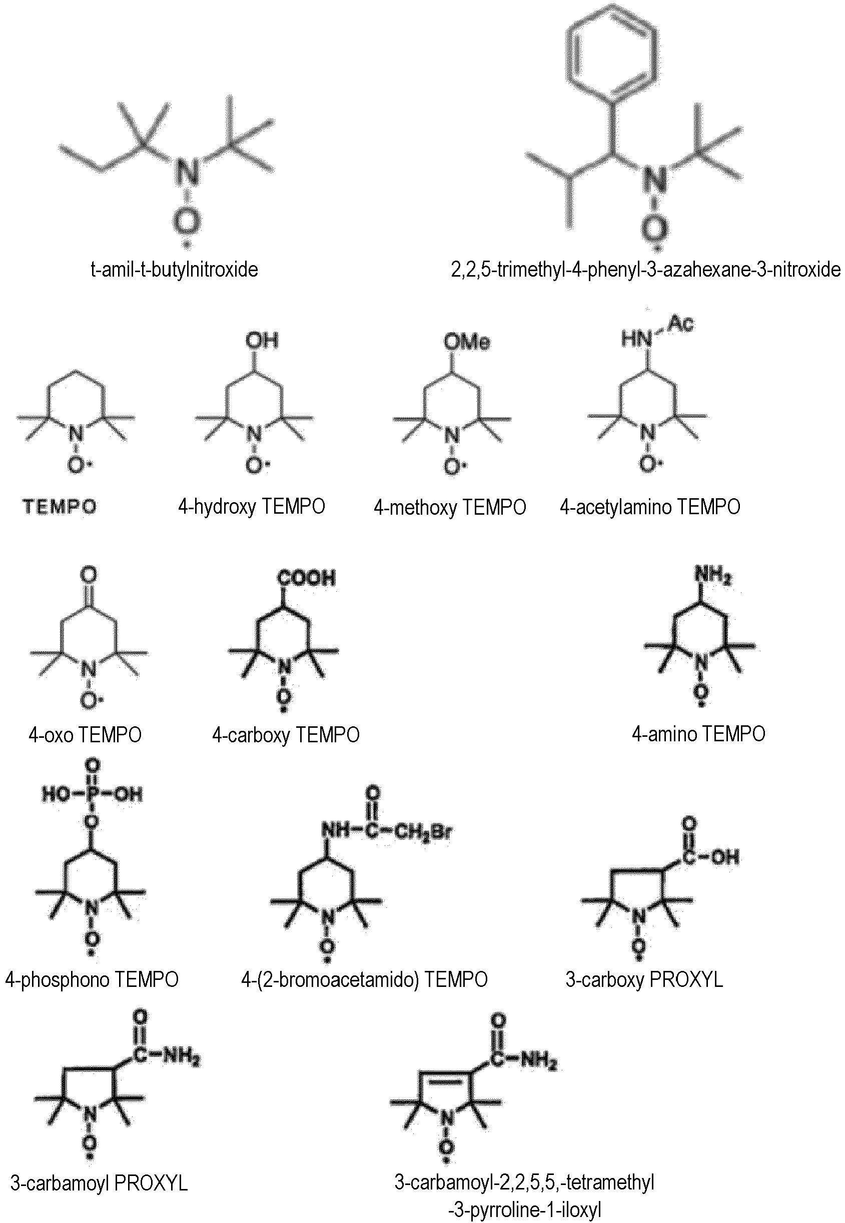

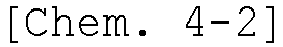

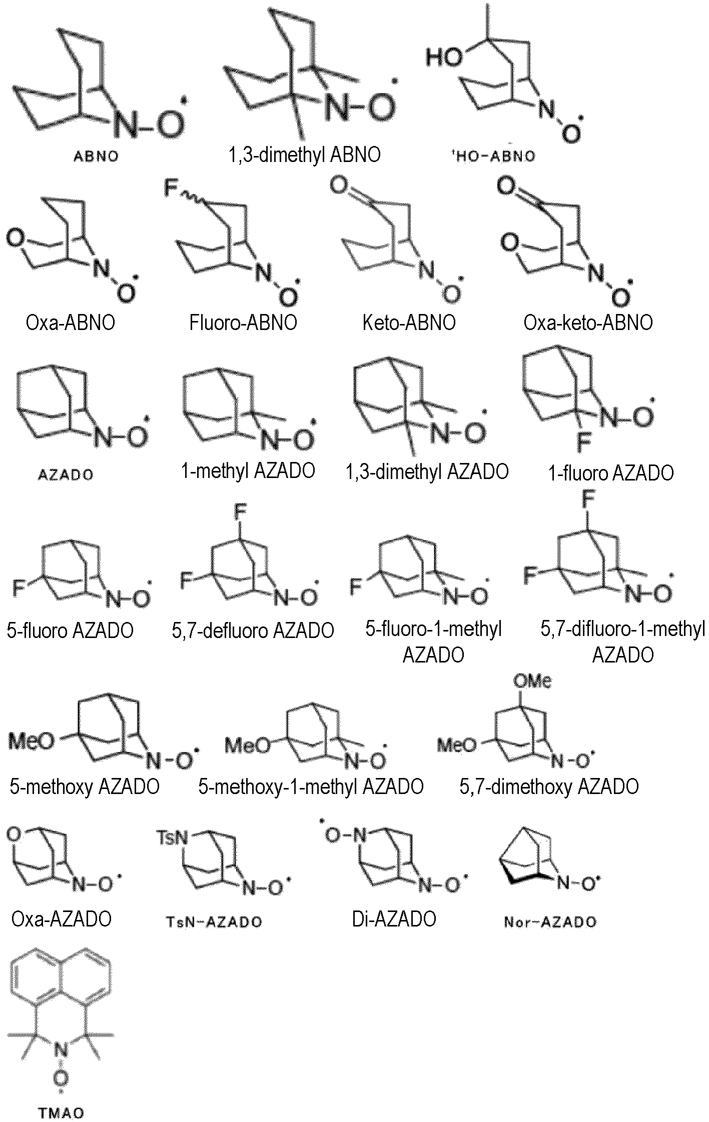

9. The method for inactivating bacteria or viruses according to claim 8, wherein the nitroxyl radical form of the catalyst is 2-azaadamantane-N-oxyl (AZADO), 2,2,6,6-tetramethylpiperidine-N-oxyl (TEMPO), or 4-acetylamino TEMPO.

10. The method for inactivating bacteria or viruses according to claim 1 or 2, wherein the compound is brought into contact with the target in the presence of a cocatalyst containing a transition metal.

11. The method for inactivating bacteria or viruses according to claim 10, wherein the transition metal contains one or more selected from a group consisting of Ag, Au, Pt, Pd, Ni, Mn, Fe, Ti, Al, Zn, and Cu.

12. The method for inactivating bacteria or viruses according to claim 1 or 2, wherein the compound is brought into contact with the target by spraying a solution containing the compound onto the target.

13. An agent for inactivating bacteria or viruses comprising a compound having a redox mechanism below: 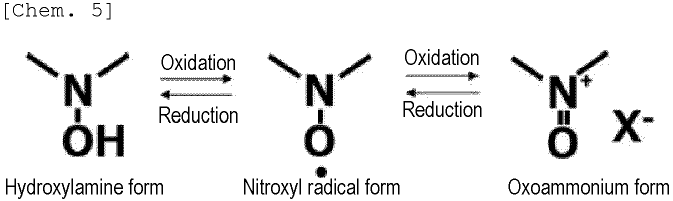 in the formula, X⁻ represents a counteranion, and a broken line represents a bonding position with another atom.

14. The agent for inactivating bacteria or viruses according to claim 13, wherein the agent is an agent for inactivating bacteria, and the bacteria are either bacterial or fungal.

15. An antimicrobial or antiviral base material, wherein a base material is supported or coated with the agent for inactivating bacteria or viruses according to claim 13 or 14.

16. The antimicrobial or antiviral base material according to claim 15, wherein the base material is further supported or coated with a cocatalyst containing a transition metal.

17. The antimicrobial or antiviral base material according to claim 15 or 16, wherein the base material is a woven, nonwoven, filter, or urethane foam.

18. The antimicrobial or antiviral base material according to claim 17, wherein the antiviral activity value (Mv) against influenza virus or feline calicivirus measured based on JIS L 1922:2016 (antiviral test method for textile products) is 2.0 or more.

19. The antimicrobial or antiviral base material according to claim 15 or 16, which is used in a mask, a medical textile product, an air conditioner, an air purifier, a refrigerator, a temperature controller, a dehumidifier, a humidifier or a filter for a vacuum cleaner.
